# EUROPEAN PATENT APPLICATION

(11) **EP 4 729 539 A1**
(43) Date of publication of application: **22.04.2026**
(21) Application number: 24822814.0
(22) Date of filing: 14.06.2024
(51) Int. Cl.: C07K 16/24, A61K 39/395, A61P 37/00, A61P 1/04

(54) **USE OF RECOMBINANT ANTI-INTERLEUKIN 23P19 SUBUNIT ANTIBODY IN TREATING INFLAMMATORY BOWEL DISEASE**

(30) Priority: 16.06.2023 CN 202310717065; 13.06.2024 CN 202410759003
(71) Applicant: Innovent Biologics (Suzhou) Co., Ltd., Suzhou, Jiangsu 215123 (CN)
(72) Inventor: QIAN, Lei, Suzhou, Jiangsu 215123 (CN); CAI, Chenghang, Suzhou, Jiangsu 215123 (CN); ZHENG, Shirui, Suzhou, Jiangsu 215123 (CN); YAN, Shuling, Suzhou, Jiangsu 215123 (CN)
(74) Representative: Sagittarius IP
(86) International application number: PCT/CN2024/099327
(87) International publication number: WO 2024/255867

(57) **Abstract**

The present invention relates to a method for treating inflammatory bowel disease, including ulcerative colitis and Crohn's disease, with a recombinant anti-IL-23p19 subunit antibody, and in particular to a dosage regimen for preventing or treating mild ulcerative colitis and moderate to severe ulcerative colitis with an anti-IL-23p19 antibody.

## Description

### FIELD OF THE INVENTION

The present invention generally relates to a treatment of inflammatory bowel diseases, including ulcerative colitis (e.g., mild or moderately severe ulcerative colitis) and Crohn's diseases, with anti-IL antibodies, and particularly to dosage regimens for the treatment of such diseases.

### Background of the Invention

Inflammatory bowel disease (IBD) is an idiopathic inflammatory bowel disease involving the ileum, rectum, and colon, and clinically manifests as diarrhea, abdominal pain, and even bloody stools. Inflammatory bowel diseases include ulcerative colitis (UC) and Crohn's disease (CD). Ulcerative colitis (UC) is a chronic inflammatory disease characterized by inflammation of the colon and rectum. The typical symptoms are recurrent diarrhea, mucopurulent bloody stools with abdominal pain, and tenesmus. UC is a serious threat to physical health and has an impact on daily life and psychology. The clinical classification of UC can be divided into primary and chronic relapsing forms. UC is more common in Europe and North America, and the incidence in China is lower than that in Western populations. However, the number of patients seeking medical treatment has been on a rapid upward trend in the past 20 years, with a prevalence rate of 11.6/100,000, and the disease is more common in young and middle-aged people. Crohn's disease (CD) is a chronic inflammatory granulomatous disease that can involve the entire digestive tract. It is a non-continuous full-thickness inflammation that most commonly involves the terminal ileum and adjacent colon. The main lesion is gastrointestinal ulcer, which can cause intestinal stenosis, perforation, and the like, and is prone to high-risk recurrence. The incidence of Crohn's disease has increased significantly over the past decade.

The clinical treatment of UC is mainly based on drug and surgical treatment. Thiopurine drugs are the most traditional immunosuppressants, but the incidence of adverse reactions is high. In terms of biological therapy, anti-TNF-α monoclonal antibodies are used for patients who are ineffective or hormone-dependent or intolerant to hormones and immunosuppressants. Infliximab (IFX) was previously the only biologics approved in China for the treatment of UC and CD. In May 2019, Ustekinumab (STELARA^{®}) was included in the second batch of urgently needed overseas new drugs list by the NMPA for the treatment of adult patients with moderately to severely active Crohn's disease (CD) who have inadequate response, loss of response, or intolerance to conventional therapy or tumor necrosis factor α (TNF-α) antagonists. In Mar. 2020, Vedolizumab (Entyvio^{®}) was approved by the NMPA for the treatment of adult patients with moderately to severely active UC and CD who have inadequate response, loss of response, or intolerance to conventional therapy or tumor necrosis factor α (TNF-α) inhibitors in China. Therefore, a variety of biologics targeting specific immune pathways have been investigated as potential therapeutic agents for UC. Anti-tumor necrosis factor-α (anti-TNFα) monoclonal antibodies and more recently the integrin receptor antagonist Vedolizumab have been approved by the US Food and Drug Administration and the European Medicines Agency.

IL-23 (interleukin-23) is a heterodimeric cytokine of IL-12 family members, and has the same p40 subunit and unique p19 subunit as IL-12. The IL-23 heterodimer activates signaling pathways by binding to the IL-23 receptor (IL-23R) and the β1 subunit of the IL-12 receptor (IL-12Rβ1). Although IL-23 is closely related to IL-12, these two cytokines play different biological roles, with IL-12 and IL-23 driving Th1 and Th17 cellular responses, respectively. Naive CD4+ T cells can be induced to differentiate into Th17 cells either alone or with the help of other cytokines such as transforming growth factor (TGF-β), IL-6 or IL-1β, and can promote Th17 and innate immune cells proliferation, differentiation and maintenance. Th17 cell stimulation produces cytokines including IL-17, IL-22, tumor necrosis factor (TNF)-α, and granulocyte-macrophage-colony stimulating factor (GM-CSF), which stimulate local tissue inflammation and other immune-mediated pathological processes through a wide range of immune-mediated inflammatory states. Chronic inflammation resulting from dysregulation of IL-23/Th17/IL-17 responses is the pathophysiological basis for a number of autoimmune diseases including psoriasis, ulcerative colitis, Crohn's disease, rheumatoid arthritis, multiple sclerosis and asthma. Several companies are seeking to treat a number of diseases with IL-23 targeted therapy. The first biologic to demonstrate clinical benefit in autoimmune diseases was Ustekinumab, a monoclonal antibody approved by the U.S. Food and Drug Administration for the treatment of psoriasis, psoriatic arthritis, and CD. Usinumab binds to the common p40 subunit of IL-12 and IL-23; thus, it targets both cytokines and non-specifically IL-23. Blocking the IL-12 pathway may prevent Th1 cells from inducing interferon blockade of Th17-producing cells, thus potentially limiting the clinical activity of antibodies targeting p40. Agents specifically targeting the IL-23p19 subunit have shown clinical activity in psoriasis and CD (Kopp T et al., Nature, Vol. 521, No. 7551, pp. 222-226, 2015; sands BE et al., Journal of Crohn's and Colitis, Vol. 9, Suppl. No. 1, pp. S15-S16, 2015).

Traditional drugs or biologics approved for the treatment of ulcerative colitis all have insufficient response rate, loss of response, or intolerance to the treatment, and have different types and degrees of adverse reactions.

### SUMMARY OF THE INVENTION

The inventors have surprisingly found that by administering the anti-IL-23p19 antibody, particularly according to the administration regimen of the present invention, in patients with inflammatory bowel disease, including ulcerative colitis and Crohn's disease a satisfactory clinical response rate and clinical remission rate were achieved, a positive therapeutic effect was obtained, no significant or unacceptable adverse reactions were observed, the safety was acceptable and controllable, and with good tolerance and good patient compliance.

The present invention also meets the above need by providing a method for treating ulcerative colitis using an anti-IL-23p19 antibody.

Accordingly, in a first aspect, provided is a method for preventing or treating inflammatory bowel disease, e.g., ulcerative colitis or Crohn's disease, in a patient, which comprises administering to the patient in need thereof the anti-IL-23p19 antibody of the present invention.

In a second aspect, provided is a use of the anti-IL-23p19 antibody of the present invention in preventing or treating inflammatory bowel disease, e.g., ulcerative colitis or Crohn's disease.

In a third aspect, provided is the anti-IL-23p19 antibody of the present invention for use in preventing or treating inflammatory bowel disease, e.g., ulcerative colitis or Crohn's disease.

In a fourth aspect, provided is a use of the anti-IL-23p19 antibody of the present invention in the manufacture of a medicament for preventing or treating inflammatory bowel disease, such as ulcerative colitis or Crohn's disease.

In a fifth aspect, provided is a pharmaceutical composition comprising an effective amount of the anti-IL-23p19 antibody of the present invention and one or more pharmaceutically acceptable excipients.

In a sixth aspect, provided is a kit of parts comprising an effective amount of the anti-IL-23p19 antibody of the present invention and a package insert with instructions for use of the anti-IL-23p19 antibody in the prevention or treatment of inflammatory bowel disease, e.g., ulcerative colitis or Crohn's disease.

In a seventh aspect, provided is a single dosage unit comprising an effective amount of the anti-IL-23p19 antibody of the present invention.

In an eighth aspect, provided is a use of the kit of parts in the manufacture of a medicament for preventing or treating inflammatory bowel disease, such as ulcerative colitis or Crohn's disease.

In one embodiment of the present invention, a method for preventing or treating inflammatory bowel disease is provided. Preferably, the inflammatory bowel disease is ulcerative colitis (e.g., mild, moderate or severe ulcerative colitis) or Crohn's disease.

In one embodiment of the present invention, a method for preventing or treating ulcerative colitis is provided. Preferably, the ulcerative colitis is moderate to severe ulcerative colitis.

In one embodiment of the present invention, the method comprises administering to a patient in need an anti-IL-23p19 antibody, wherein the administration regimen comprises:
a) an induction treatment phase: administering one or more induction doses of the anti-IL-23p19 antibody to a patient in need thereof, wherein each the induction doses is 100-1000 mg; and
b) a maintenance treatment phase: administering to the patient one or more maintenance doses of the anti-IL-23p19 antibody, the first maintenance dose being administered 2-8 weeks after the last induction dose is administered, wherein each maintenance doses is 100-1000 mg, wherein the amino acid sequence of the heavy chain CDR1 of the antibody comprises or consists of SEQ ID NO. 1, the amino acid sequence of the heavy chain CDR2 comprises or consists of SEQ ID NO. 2, and the amino acid sequence of the heavy chain CDR3 comprises or consists of SEQ ID NO. 3; the amino acid sequence of the light chain CDR1 of the antibody comprises or consists of SEQ ID NO. 4, the amino acid sequence of the light chain CDR2 comprises or consists of SEQ ID NO. 5, and the amino acid sequence of the light chain CDR3 comprises or consists of SEQ ID NO. 6.

In one embodiment of the present invention, the induction dose is administered by intravenous injection, and/or the maintenance dose is administered by subcutaneous injection.

In one embodiment of the invention, administration of the induction dose produces a clinical response and the patient enters a maintenance treatment phase.

In one embodiment of the present invention, the induction dose is administered 1-3 times, preferably 3 times.

In one embodiment of the invention, when multiple induction doses are administered, the induction doses are administered at 4-week intervals.

In one embodiment of the invention, when multiple maintenance doses are administered, the maintenance doses are administered at intervals of 4-8 weeks.

In one embodiment of the invention, the first maintenance dose is administered 2-8 weeks after the administration of the last induction dose. Preferably, the first maintenance dose is administered 4 weeks after administration of the last induction dose.

In one embodiment of the present invention, the method comprises administering to a patient in need an anti-IL-23p19 antibody, wherein the administration regimen comprises:
a) an induction treatment phase: administering to a patient in need thereof 3 induction doses of an anti-IL-23p19 antibody at 4-week intervals, wherein each induction dose is 100-1000 mg;
b) a maintenance treatment phase: administering to the patient maintenance doses of the anti-IL-23p19 antibody at intervals of 4-8 weeks, the first maintenance dose being administered 2-8 weeks after the last induction dose, wherein each maintenance dose is 100-1000 mg;
wherein the amino acid sequence of the heavy chain CDR1 of the antibody comprises or consists of SEQ ID NO. 1, the amino acid sequence of the heavy chain CDR2 comprises or consists of SEQ ID NO. 2, and the amino acid sequence of the heavy chain CDR3 comprises or consists of SEQ ID NO. 3; the amino acid sequence of the light chain CDR1 of the antibody comprises or consists of SEQ ID NO. 4, the amino acid sequence of the light chain CDR2 comprises or consists of SEQ ID NO. 5, and the amino acid sequence of the light chain CDR3 comprises or consists of SEQ ID NO. 6, and the boundaries of the CDRs are determined by AbM scheme.

In one embodiment of the present invention, the ulcerative colitis is moderate to severe ulcerative colitis. In another embodiment, the ulcerative colitis is mild ulcerative colitis.

In one embodiment of the invention, the induction dose is administered by intravenous injection. Preferably, the induction dose is administered by intravenous infusion.

In one embodiment of the invention, the maintenance dose is administered by subcutaneous injection.

In one embodiment of the present invention, 3 induction doses of the anti-IL-23p19 antibody are administered to a patient in need thereof by intravenous infusion at intervals of 4 weeks.

In one embodiment of the present invention, a maintenance dose of the anti-IL-23p19 antibody is administered to a patient in need thereof by subcutaneous injection at intervals of 4 weeks.

In one embodiment of the present invention, a maintenance dose of the anti-IL-23p19 antibody is administered to a patient in need thereof by subcutaneous injection at intervals of 8 weeks.

In one embodiment of the invention, the induction dose in the method is 100 mg, 200 mg, 300 mg, 400 mg, 500 mg, 600 mg, 700 mg, 800 mg, 900 mg, or 1000 mg.

In one embodiment of the invention, the induction dose in the method is 200 mg.

In one embodiment of the invention, the induction dose in the method is 600 mg.

In one embodiment of the invention, the method wherein a maintenance dose is administered at 4-week intervals after administration of the first maintenance dose.

In one embodiment of the invention, the first maintenance dose in the method is for administration 4 weeks after administration of the last induction dose.

In one embodiment of the invention, the maintenance dose in the method is 100 mg, 200 mg, 300 mg, 400 mg, 500 mg, 600 mg, 700 mg, 800 mg, 900 mg or 1000 mg.

In one embodiment of the invention, the maintenance dose in the method is 200 mg.

In one embodiment of the present invention, the administration regimen in the method comprises an extended induction phase: administering to a patient in need thereof 3 induction doses of the anti-IL-23p19 antibody at 4-week intervals, and if the patient does not achieve a clinical response 2-8 weeks after the last induction dose is administered, 3 extended induction doses are administrated to the patient, each extended induction dose being 100-1000 mg; if the patient produces clinical response to the administration of the extended induction dose, a maintenance treatment phase is entered.

In one embodiment of the invention, wherein no clinical response is achieved 4 weeks after the last induction dose is administered, the patient is administrated 3 extended induction doses, each extended induction dose being 100-1000 mg .

In one embodiment of the invention, the extended induction dose is administered at 4 week intervals.

In one embodiment of the invention, the extended induction dose is administered by intravenous infusion.

In one embodiment of the invention, the first extended induction dose is administered 4 weeks after the last induction dose is administered.

In one embodiment of the invention, the extended induction dose in the method is 100 mg, 200 mg, 300 mg, 400 mg, 500 mg, 600 mg, 700 mg, 800 mg, 900 mg, or 1000 mg.

In one embodiment of the invention, the extended induction dose in the method is 600 mg.

In one embodiment of the invention, after the extended induction phase, the first maintenance dose is administered 2-8 weeks after the last extended induction dose is administered.

In one embodiment of the invention, after the extended induction phase, the first maintenance dose is administered 4 weeks after the last extended induction dose is administered.

In one embodiment of the present invention, a maintenance dose of the anti-IL-23p19 antibody is administered to a patient in need thereof by subcutaneous injection at 4-week intervals after an extended induction phase. In a further embodiment, the maintenance dose is 100-1000 mg.

In one embodiment of the invention, the method comprises administering a maintenance dose after the extended induction phase, wherein each maintenance dose is 100 mg, 200 mg, 300 mg, 400 mg, 500 mg, 600 mg, 700 mg, 800 mg, 900 mg, or 1000 mg.

In one embodiment of the invention, the method comprises administering a maintenance dose after the extended induction phase, wherein each maintenance dose is 200 mg.

The method of the present invention comprises administering to a patient in need thereof at least one induction dose of the anti-IL-23p19 antibody in the induction phase to induce a desired therapeutic effect, wherein the desired therapeutic effect is clinical remission, clinical response, endoscopic remission, mucosal healing, and/or symptomatic remission. If the patient achieves the desired therapeutic effect at the end of the induction phase, he/she is subsequently administered at least one maintenance dose to maintain the at least one therapeutic effect achieved during the induction phase which is clinical remission, clinical response, endoscopic remission, mucosal healing, and/or symptomatic remission.

There is no minimum or maximum duration of the induction phase, but it is typically a period of 4 weeks, 8 weeks or 12 weeks, and the end of the induction phase is an end of induction assessment conducted 4 or 8 weeks after the last induction dose has been administered. For example, a 4-week induction phase may include administration of an induction dose at week 0 and an end of induction assessment at week 4. An 8-week induction phase may include administration of an induction dose at weeks 0 and 4 and an end of induction assessment at week 8. The 12-week induction phase may include administration of an induction dose at weeks 0, 4 and 8 and an end of induction assessment at week 12.

In one embodiment of the present invention, the heavy chain of the anti-IL-23p19 antibody comprises an N-glycosylation site located at asparagine 295 of the heavy chain.

In one embodiment of the present invention, the anti-IL-23p19 antibody in the method comprises a heavy chain variable region VH and a light chain variable region VL, wherein the heavy chain variable region comprises or consist of SEQ ID NO:7 or a sequence having at least 90%, 95%, 98% or 99% identity thereto, and the light chain variable region comprises or consists of SEQ ID NO:8 or a sequence having at least 90%, 95%, 98% or 99% identity thereto.

In one embodiment of the present invention, the anti-IL-23p19 antibody in the method is an IgG1 antibody, preferably comprising a heavy chain and a light chain, wherein the heavy chain comprises or consists of SEQ ID NO: 9 or a sequence having at least 90%, 95%, 98%, or 99% identity thereto, and the light chain comprises or consist of SEQ ID NO:10 or a sequence having at least 90%, 95%, 98% or 99% identity thereto.

In one embodiment of the present invention, the anti-IL-23p19 antibody comprises a heavy chain set forth in SEQ ID NO: 9 and a light chain set forth in SEQ ID NO: 10; for example, the anti-IL-23p19 antibody comprises or consists of 2 heavy chains set forth in SEQ ID NO: 9 and 2 light chains set forth in SEQ ID NO: 10.

In one embodiment of the present invention, the anti-IL-23p19 antibody is recombinantly expressed in HEK 293 cells or CHO cells.

In one embodiment of the present invention, the present invention also relates to a kit of parts, comprising the effective amount of the anti-IL-23p19 antibody described above; preferably, the kit of parts further comprises a package insert with instructions for using the anti-IL-23p19 antibody to prevent or treat inflammatory bowel disease, such as ulcerative colitis or Crohn's disease, in an individual.

The present invention also provides a single dosage unit comprising: an effective amount of the anti-IL-23p19 antibody of the present invention, and a kit of parts comprising an effective amount of the anti-IL-23p19 antibody of the present invention.

In one embodiment of the present invention, further included is use of the kit of parts in the manufacture of a medicament for preventing or treating inflammatory bowel disease, such as ulcerative colitis or Crohn's disease.

The method for preventing or treating inflammatory bowel disease, e.g., ulcerative colitis or Crohn's disease, in an individual with the anti-IL-23p19 antibody of the present invention is administered once every 4 or 8 weeks in the maintenance phase, which can increase the patient's compliance with the medicament, reduce the patient's fear of injection, reduce the patient's physical and psychological burden, and help maintain the patient's long-term remission.

In yet another embodiment of the method of the invention, the patient is biological drug-naive.

In an alternative embodiment of the method of the invention, the patient has undergone biopharmaceutical treatment.

This embodiment of the method of the invention comprises administering one, two or three further induction dose(s) - referred to as "extended induction dose(s)" - to distinguish it from the initial induction dose if the patient does not achieve a clinical response at the end of the initial induction phase. The dose and dosing interval during the extended induction phase will generally be the same as the dose and dosing interval during the initial induction phase, but may be changed if the attending health care professional has reason to believe that the patient may benefit from a change, such as an increased dose or more frequent administration of the anti-IL-23p19 antibody. If the patient achieves a clinical response at the end of the extended induction phase, at least one maintenance dose of the anti-IL-23p19 antibody is administered to maintain clinical response or other desired therapeutic effect, such as clinical remission, endoscopic remission, mucosal healing, and/or symptomatic remission. The first maintenance dose is administered 4-12 weeks after the last extended induction dose is administered to the patient. This 4-12 week period accounts for the time change between administration of the last extended induction dose and the end of extended induction assessment. The change may result from a change in dosing frequency during an extended induction phase. For example, the dosing frequency is every 4 weeks during the extended induction phase and the end of extended induction assessment is made 4 weeks after the last extended induction dose is administered. If the patient has achieved clinical response, the first maintenance dose may be administered at the end of induction assessment visit (i.e., 4 weeks after the last extended induction dose is administered) or may be administered at a subsequent visit planned shortly thereafter. Alternatively, the dosing frequency is every 8 weeks during the extended induction phase and the end of extended induction assessment is made 8 weeks after the last extended induction dose is administered. If the patient has achieved clinical remission, the first maintenance dose may be administered at the end of induction assessment visit (i.e., 8 weeks after the last extended induction dose is administered) or may be administered at a subsequent visit scheduled shortly thereafter.

Other embodiments of the present invention will be apparent from and elucidated with reference to the following detailed description.

### DETAILED DESCRIPTION OF THE INVENTION

Before describing the present invention in detail, it is to be understood that this invention is not limited to the particular methods and experimental conditions described herein, as such methods and conditions may vary. Additionally, the terminology used herein is for the purpose of describing particular embodiments only and is not intended to be limiting.

### DEFINITIONS

Unless defined otherwise, all technical and scientific terms used herein have the same meaning as commonly understood by those of ordinary skill in the art. For the purposes of the present invention, the following terms are defined below.

The term "about", when used in combination with a numerical value, is intended to encompass the numerical values in a range from a lower limit less than the specified numerical value by 5% to an upper limit greater than the specified numerical value by 5%.

The terms "and/or", when used to connect two or more options, should be understood to refer to any one of the options or any two or more of the options.

As used herein, the term "comprise" or "include" is indented to include the described elements, integers or steps, but not to exclude of any other elements, integers or steps. As used herein, the term "comprise" or "include", unless indicated otherwise, also encompasses the situation where the entirety consists of the described elements, integers or steps.For example, when referring to an antibody variable region "comprising" a particular sequence, it is also intended to encompass an antibody variable region consisting of that particular sequence.

The p19 subunit of IL-23 (also referred to herein as"IL-23p19" or "p19 subunit") is a polypeptide having 189 amino acids, which contains a leader sequence having 21 amino acids(Oppmann et al., Immunity 13:715(2000), SEQ ID NO: 181), and comprises 4 compressed(packed) αhelices referred to as A, B, C and D, with an upper-upper-lower-lower topology. The 4 helices are linked by 3 polypeptide loops. The A-B and C-D loops are made relatively long because they link parallel helices. The short B-C loop links the antiparallel B and C helices. The p19 subunit of IL-23 is a member of the IL-6 family of helical cytokines. This cytokine family binds to its cognate receptors via 3 conserved epitopes(sites I, II and III; Bravo and Heath (2000) EMBO J. 19:2399-2411). The p19 subunit interacts with 3 cytokine receptor subunits to form a competent signaling complex. When expressed in a cell, the p19 subunit first forms a complex with the p40 subunit, and the p19 subunit shares the p40 subunit with IL-12. The p19p40 complex is secreted from cells as a heterodimeric protein and is referred to as IL-23. In one embodiment, the IL-23p19 of the present invention is derived from human(NCBI:AAG37232)or cynomolgus monkey(NCBI:AEY84629).

The term "anti-IL-23p19 antibody", "anti-IL-23p19", "IL-23p19 antibody" or " antibody that binds to IL-23p19" as used herein refers to an antibody that is capable of binding to the(human or cynomolgus monkey) IL-23p19 subunit or a fragment thereof with sufficient affinity such that the antibody can be used as a diagnostic and/or therapeutic agent targeting(human or cynomolgus monkey)IL-23p 19.

As used herein, the term "antibody" is used in the broadest sense, and refers to a protein comprising an antigen-binding site, and encompasses natural and artificial antibodies with various structures, including but not limited to intact antibodies and antigen-binding fragments of antibodies.

The terms "whole antibody", "full-length antibody", "complete antibody "and" intact antibody" are used interchangeably herein to refer to a glycoprotein comprising at least two heavy chains(H)and two light chains(L)interconnected by disulfide bonds. Each heavy chain consists of a heavy chain variable region (abbreviated herein as VH) and a heavy chain constant region. The heavy chain constant region consists of 3 domains, CH1, CH2 and CH3. Each light chain consists of a light chain variable region (abbreviated herein as VL) and a light chain constant region. The light chain constant region consists of one domain CL. The VH region and VL region can be further subdivided into hypervariable regions (complementarity determining regions(CDRs), with more conserved regions (framework regions (FRs)) interposed therebetween. Each VH and VL consists of three CDRs and 4 FRs, arranged from amino-terminus to carboxyl-terminus in the following order: FR1, CDR1, FR2, CDR2, FR3, CDR3 and FR4. The constant region is not directly involved in the binding of an antibody to an antigen, but exhibits various effector functions.

"Complementarity Determining Region" or "CDR region" or "CDR" is a region in an antibody variable domains that is hypervariable in sequence and form structurally defined loops ("hypervariable loops") and/or contain antigen-contacting residues ("antigen-contacting sites" ). The CDRs are primarily responsible for binding to antigenic epitopes. The CDRs of the heavy and light chains are generally referred to as CDR1, CDR2 and CDR3, and are numbered sequentially from the N-terminus. The CDRs located in the heavy chain variable domain of an antibody are referred to as HCDR1, HCDR2 and HCDR3, and the CDRs located in the light chain variable domain of an antibody are referred to as LCDR1, LCDR2 and LCDR3.

In a given amino acid sequence of a light chain variable region or a heavy chain variable region, the exact amino acid sequence boundary of each CDR can be determined using any one or a combination of many well-known antibody CDR assignment systems including, for example, Chothia based on the three-dimensional structure of antibodies and the topology of the CDR loops (Chothia et al.. (1989) Nature 342:877-883, Al-Lazikani et al., "Standard conformations for the canonical structures of immunoglobulins", Journal of Molecular Biology, 273, 927-948 (1997)), Kabat based on antibody sequence variability (Kabat et al., Sequences of Proteins of Immunological Interest, 4th edition, U.S. Department of Health and Human Services, National Institutes of Health (1987)), AbM (University of Bath), Contact (University College London), International ImMunoGeneTics database (IMGT) (imgt.cines.fr/ on the world wide web), and the North CDR definition based on the affinity propagation clustering using a large number of crystal structures.

An exemplary AbM scheme is as follows:

| CDR | AbM scheme |
|---|---|
| LCDR1 | L24-L34 |
| LCDR2 | L50-L56 |
| LCDR3 | L89-L97 |
| HCDR1 | H26-H35B |
| HCDR2 | H50-H58 |
| HCDR3 | H95-H102 |

Unless otherwise stated, in the present disclosure, when referring to residue positions in an antibody variable region (including heavy chain variable region residues and light chain variable region residues), it means according to the Kabat numbering system(Kabat et al., Sequences of Proteins of Immunological Interest, 5th Ed. Public Health Service, National Institutes of Health, Bethesda, Md.(1991)).

In one embodiment, the boundaries of the CDRs of the antibody of the present invention are determined by the AbM scheme.

"Antibody fragment" refers to a molecule different from an intact antibody, which comprises a portion of the intact antibody and binds to an antigen to which the intact antibody binds. Examples of antibody fragments include, but are not limited to, Fv, Fab, Fab', Fab'-SH, F(ab')2; diabodies; linear antibodies; single-chain antibodies(e.g., scFv); single domain antibodies; bivalent or bispecific antibodies or fragments thereof; camelid antibodies; and bispecific or multispecific antibodies formed from antibody fragments.

The term "antigen-binding fragment" as used herein refers to a molecule different from an intact antibody, which comprises a portion of the intact antibody and binds to an antigen to which the intact antibody binds. Examples of the antigen-binding fragment include, but are not limited to, Fv, Fab, Fab', Fab'-SH, and F(ab')2; a diabody; a linear antibody; a single-chain antibody(e.g., scFv); a single-domain antibody; a bivalent or bispecific antibody or a fragment thereof; a camelid antibody; and a bispecific antibody or a multispecific antibody formed from antibody fragments.

The term "pharmaceutical composition" or "formulation" refers to a composition comprising at least one active ingredient and at least one inactive ingredient, e.g., a pharmaceutically acceptable excipient, which is suitable for administration to animals, preferably mammals including humans. The pharmaceutical compositions of the present invention are preferably in the form of parenteral administration, e.g., in the form of solutions, suspensions, lyophilized powders, concentrated solutions and the like.

The term "kit of parts" refers to a kit comprising one or more separate pharmaceutical compositions, at least one of which comprises an effective amount of an anti-IL antibody, together with a package insert with instructions for use of the anti-IL-23p19 antibody in the prevention or treatment of inflammatory bowel disease, e.g., ulcerative colitis or Crohn's disease, in particular according to the administration regimen of the present invention. The kit of parts may also comprise other therapeutic agents having the same or different activities. It will be appreciated that where a kit of parts comprises a plurality of separate pharmaceutical compositions, each of the pharmaceutical compositions may comprise different doses and/or be administered by different routes.

As used herein, the term "single pharmaceutical dosage unit "refers to a single pharmaceutical dosage form comprising the antibody of the present invention to be administered to a patient at the time of administration, such as a vial, an ampoule, a pre-filled syringe or a pre-filled syringe for injection, which contains a solution or a lyophilized powder of the drug.

The terms "patient" and "subject" are used interchangeably and refer to an animal. Preferably, the patient or subject is a mammal, such as a primate(e.g., human, monkey, or chimpanzee), cow, sheep, goat, horse, dog, cat, rabbit, rat, mouse, etc. More preferably, the patient or subject is a human.

The term "biological drug-naive" patient or subject refers to a patient or subject who has not previously received a biologics treatment, including, for example, anti-tumor necrosis factor-α (TNF-α) antagonists such as adalimumab and infliximab, anti-integrin antibodies such as vedolizumab, or other experimental biological treatments.

The term "parenteral administration" as used herein means modes of administration other than enteral and topical administration, usually by injection or infusion, and includes, without limitation, intravenous, intramuscular, intraarterial, intrathecal, intracapsular, intraorbital, intracardiac, intradermal, intraperitoneal, transtracheal, subcutaneous, subcuticular, intra-articular, subcapsular, subarachnoid, intraspinal, epidural and intrasternal injection and infusion.

The term "pharmaceutically acceptable" refers to that the substances, compositions, dosage forms, and the like described hereinafter are free from excessive toxicity, irritation, allergic response, and other undesirable properties to mammals, especially humans, and are commensurate with a reasonable benefit/risk ratio when applied to animals or humans.

The term "pharmaceutically acceptable excipient" refers to an ingredient in a pharmaceutical formulation, other than an active ingredient, which is non-toxic to an individual. Examples of the pharmaceutically acceptable carrier include, but are not limited to, binders, disintegrants, lubricants, solvents, dispersion media, buffers, excipients, antioxidants, preservatives, flavoring agents, or the like.

The term "effective amount" refers to an amount effective, at dosages and for periods of time necessary, to achieve the desired therapeutic or prophylactic effect. It may be determined by the attending physician or veterinary practitioner and will vary with the compound, the disease state being treated, the severity of the disease being treated, the age and relative health of the individual, the route and form of administration, the judgment of the attending physician or veterinary practitioner, and like factors. Generally, a "prophylactically effective amount" will be less than a "therapeutically effective amount".

The terms "treat," "treating," and "treatment" refer to alleviating or reducing the severity of at least one symptom or condition, slowing or arresting its progression, or curing the condition.

The term "preventing" refers to delaying or precluding the occurrence of a disorder or symptom thereof.

The term "inflammatory bowel disease" refers to an idiopathic intestinal inflammatory disease involving the ileum, rectum, and colon, with clinical manifestations of diarrhea, abdominal pain, and even bloody stools.

The term "ulcerative colitis" refers to a continuous inflammation of the mucosal and submucosal layers of the colon, mainly involving the rectum and colon, which can be classified into mild, moderate, and severe ulcerative colitis according to the severity of the disease. The modified Mayo scoring system can be used to assess the severity and activity of ulcerative colitis.

The term "Crohn's disease" is a chronic inflammatory granulomatous disease of unknown cause that may involve the entire digestive tract, which is a discontinuous full-thickness inflammation, and the main lesion is gastrointestinal ulcer.

The term "week 0" refers to the time point of the first administration of the IL-23p19 antibody. The term "week 4" refers to the 4th week from the time point of the first administration of the IL-23p19 antibody. The term "week 8" refers to the 8th week from the time point of the first administration of the IL-23p19 antibody. Other similar terms should also be understood accordingly.

As used herein, the "modified Mayo score" is derived from the Mayo scoring system for the severity and activity of ulcerative colitis (Schreder KW, Tremaine WJ, Ilstrup DM. Coated oral 5-aminosalicylic acid therapy for mildly to moderately active ulcerative colitis. A randomized study. N Engl J Med 1987; 317(26):16 25-9), where the physician's global assessment is not used according to the recommendations of the US Food and Drug Administration (FDA), and consists of three items of stool frequency, rectal bleeding, and endoscopic findings, with a score ranging from 0 to 9 points.

As used herein, the "symptomatic Mayo score" refers to the modified Mayo score without endoscopic findings with a score ranging from 0 to 6 points.

As used herein, "partial Mayo score" refers to the Mayo score without endoscopic findings, i.e., with defecation, rectal bleeding, and physician's global assessment. It is only used to evaluate clinical recurrence and does not used as efficacy indicators.

As used herein, "clinical remission" is the primary endpoint of the study, defined as rectal bleeding of 0 point, stool frequency of ≤1 point, and endoscopic findings of ≤1 point on the modified Mayo score.

As used herein, "clinical response" refers to a decrease from baseline in the modified Mayo score of ≥ 30% and ≥ 2 points, and a decrease from baseline in the rectal bleeding score of ≥ 1 points or a subscore of 0 or 1.

As used herein, "symptomatic remission" refers to a stool frequency score of 0 or 1 and a rectal bleeding score of 0.

As used herein, "endoscopic remission" refers to an endoscopic score of 0 or 1 in the modified Mayo score. An endoscopic score of 0 was considered complete endoscopic remission.

As used herein, "mucosal healing" refers to endoscopic remission with histopathologically centered Geboes index remission.

As used herein, an "induction dose" refers to the first dose of an anti-IL-23p19 antibody administered to a patient in order to induce clinical remission, clinical response, endoscopic remission, mucosal healing, and/or symptomatic remission. An "induction dose" may be a single administration. The "induction dose" may also be a multiple administration. Reference herein to an "induction dose" generally refers to the dose administered during the induction treatment phase, unless otherwise indicated or clearly contradicted by context.

As used herein, an "induction treatment phase" refers to a phase in which an "induction dose" is administered for the purpose of inducing clinical remission, clinical response, endoscopic remission, mucosal healing, and/or symptomatic remission.

As used herein, "extended induction phase" refers to a phase of treating a patient that includes administering an anti-IL-23p19 antibody to the patient for the purpose of inducing clinical remission, clinical response, endoscopic remission, mucosal healing, and/or symptomatic remission because clinical remission, clinical response, endoscopic remission, mucosal healing, and/or symptomatic remission were not achieved during the initial induction treatment phase. The "extended induction phase" may be during 4, 8 or 12 weeks.

As used herein, "extended induction dose" refers to an additional induction dose of an anti-IL-23p19 antibody, wherein the additional induction dose is administered to a patient with the intent to induce clinical remission, clinical response, endoscopic remission, mucosal healing, and/or symptomatic remission because clinical remission, clinical response, endoscopic remission, mucosal healing, and/or symptomatic remission are not achieved during the initial induction phase. The "extended induction dose" may be a single dose or alternatively a set of doses. There is no minimum or maximum duration for an extended induction phase, but it is typically during 4 weeks, 8 weeks or 12 weeks. The extended end-of-induction phase is generally an extended end-of-induction assessment performed 4 or 8 weeks after the last extended induction dose has been administered. An "extended induction dose" is administered during an extended induction treatment phase.

As used herein, "maintenance (treatment) phase" refers to a treatment phase that comprises administering an anti-IL-23p19 antibody to a patient to maintain a desired therapeutic effect, which is clinical remission, clinical response, endoscopic remission, mucosal healing, and/or symptomatic remission. The "maintenance phase" follows the induction treatment phase or extended induction treatment phase, and is thus initiated once the desired therapeutic effect-clinical remission, clinical response, endoscopic remission, mucosal healing, and/or symptomatic remission-is achieved.

As used herein, "maintenance dose" refers to a subsequent dose of the anti-IL-23p19 antibody, wherein the subsequent dose is administered to maintain or continue the desired therapeutic effect, i.e., clinical remission, clinical response, endoscopic remission, mucosal healing, and/or symptomatic remission. The "maintenance dose" is administered after the induction dose. A "maintenance dose" may be a single administration or alternatively a set of administrations. The dose of the first administration in the maintenance phase subsequent to the end of the induction phase administration is referred to as the "first maintenance dose".

In some embodiments, the administration regimen of the present invention comprises an induction treatment phase followed by a maintenance treatment phase, wherein the induction treatment phase and maintenance treatment phase are as described herein.

In other embodiments, the administration regimen of the present invention comprises an induction treatment phase, followed by an extended induction phase, and followed by a maintenance treatment phase, wherein the induction treatment phase, the extended induction phase, and the maintenance treatment phase are as described herein.

In some embodiments, the Mayo score, preferably the modified Mayo score, is performed at the end of the induction treatment phase. In particular, the Mayo score, preferably the modified Mayo score, is performed at weeks 11-12.

In some embodiments, the Mayo score, preferably the modified Mayo score, is performed at the end of the extended induction phase. In particular, the Mayo score, preferably the modified Mayo score, is performed at weeks 23-24.

In some embodiments, the safety follow-up is performed for 8 weeks after the last maintenance dose.

In some embodiments, the administration regimen of the present invention comprises:
a) an induction treatment phase: administering one or more induction doses of the anti-IL-23p19 antibody to a patient in need thereof, wherein each the induction doses is 100-1000 mg; and
b) a maintenance treatment phase: administering to the patient one or more maintenance doses of the anti-IL-23p19 antibody, the first maintenance dose being administered 2-8 weeks after the last induction dose is administered, wherein each maintenance doses is 100-1000 mg.

In some embodiments, the induction treatment phase comprises administering to the patient one or more induction doses of the anti-IL-23p19 antibody, wherein each the induction doses is 100-1000 mg.Preferably, the induction dose is administered by intravenous injection. More preferably, the induction dose is administered by intravenous infusion.

In some embodiments, administration of the induction dose produces a clinical response and the patient enters the maintenance treatment phase.

In some embodiments, the induction dose may be administered once or multiple times, e.g., once, twice, three times, or four times. Preferably, the induction dose is administered 1-3 times. More preferably, the induction dose is administered 3 times. When multiple induction doses are administered, the induction doses are administered at 4 week intervals. It will be appreciated that the induction doses may each be equal or unequal.

In one embodiment, the patient achieves clinical response 2-8 weeks after receiving the last induction dose of the induction treatment phase and then the maintenance phase. In some embodiments, the first maintenance dose is administered 2-8 weeks after the administration of the last induction dose. Preferably, the first maintenance dose is administered 4 weeks after administration of the last induction dose.

In some embodiments, the maintenance treatment phase comprises administering to the patient one or more maintenance doses of the anti-IL-23p19 antibody, wherein each maintenance doses is 100-1000 mg. Preferably, the maintenance dose is administered by subcutaneous injection.

In one embodiment, the maintenance dose may be administered one or more times. In one embodiment, when multiple maintenance doses are administered, the maintenance doses are administered at intervals of 4-8 weeks. Preferably, the maintenance doses are administered at an interval of 4 weeks or 8 weeks. It will be appreciated that where multiple maintenance doses are administered, the maintenance doses may each be equal or unequal. In particular, the maintenance treatment phase lasts for 32 weeks from the administration of the first maintenance dose.

In a specific embodiment of the present invention, the method comprises administering to a patient in need thereof the anti-IL-23p19 antibody, wherein the administration regimen comprises: administering to the patient in need thereof 3 induction doses of the anti-IL-23p19 antibody at 4-week intervals, each induction dose being 100-1000 mg.

In a specific embodiment of the invention, the method comprises administering to the patient maintenance doses of the anti-IL-23p19 antibody at intervals of 4-8 weeks, the first maintenance dose being administered 2-8 weeks after the last induction dose is administered, each maintenance dose being 100-1000 mg.

In one embodiment, the administration regimen of the present invention comprises:
a) an induction treatment phase: administering to a patient in need thereof 3 induction doses of an anti-IL-23p19 antibody at 4-week intervals, wherein each induction dose is 100-1000 mg; and
b) a maintenance treatment phase: administering to the patient maintenance doses of the anti-IL-23p19 antibody at intervals of 4-8 weeks, the first maintenance dose being administered 2-8 weeks after the last induction dose, wherein each maintenance dose is 100-1000 mg.

In one specific embodiment of the present invention, the anti-IL-23p19 antibody is an antibody that specifically binds to IL-23p19 and comprises the following 6 CDRs:
- a heavy chain VH CDR1 comprising or consisting of GYTFTSYLMH (SEQ ID NO: 1);
- a heavy chain VH CDR2 comprising or consisting of YINPYNEGTN (SEQ ID NO: 2);
- a heavy chain VH CDR3 comprising or consisting of NWDLPY (SEQ ID NO: 3);
- a light chain VL CDR1 comprising or consisting of RASQSISDYLH (SEQ ID NO: 4);
- a light chain VL CDR2 comprising or consisting of YASQSMS (SEQ ID NO: 5); and
- a light chain VL CDR3 comprising or consisting of QQGHSFPFT (SEQ ID NO: 6).

In particular, the boundaries of the CDRs are determined by AbM scheme.

In a specific embodiment, the inflammatory bowel disease is ulcerative colitis (UC) or Crohn's disease (CD).

In a specific embodiment, the UC is moderate to severe ulcerative colitis, and the moderate to severe ulcerative colitis includes, but is not limited to, moderate to severe active ulcerative colitis (modified Mayo score 4-9 points, with an endoscopic score of ≥ 2 points). In a specific embodiment, the UC is mild ulcerative colitis.

In a specific embodiment, the method comprises administering to a patient in need thereof 3 induction doses of the anti-IL-23p19 antibody by intravenous infusion at 4-week intervals.

In one specific embodiment, the method comprises administering to a patient in need thereof a maintenance dose of the anti-IL-23p19 antibody by subcutaneous injection at intervals of 4-8 weeks, e.g., 4 weeks, 6 weeks, 7 weeks, or 8 weeks.

In a specific embodiment, the induction dose in the method is 100-1000 mg, e.g., 100, 200, 300, 400, 500, 600, 700, 800, 900, or 1000 mg. It will be appreciated that the induction dose may be 100, 200, 300, 400, 500, 600, 700, 800, 900 or 1000 mg or a range consisting of any two thereof, for example, 200-900 mg, 200-800 mg, 200-600 mg, etc. In a specific embodiment, the induction dose is 200 mg. In another specific embodiment, the induction dose is 600 mg.

In a specific embodiment, the maintenance dose may be administered one or more times. It will be appreciated that the maintenance doses may each be equal or unequal. When multiple maintenance doses are administered, the maintenance doses are administered at 2-8 week intervals. In a specific embodiment, in this method a maintenance dose is administered at an interval of 2-8 weeks, e.g., 2 weeks, 3 weeks, 4 weeks, 5 weeks, 6 weeks, 7 weeks, or 8 weeks after administration of the first maintenance dose. In particular, the maintenance doses are administered at an interval of 4 weeks or 8 weeks. In particular, the maintenance treatment phase lasts for 32 weeks from the administration of the first maintenance dose.

In a specific embodiment, the first maintenance dose in the method is for administration 2-8 weeks, e.g., 2 weeks, 3 weeks, 4 weeks, 5 weeks, 6 weeks, 7 weeks, or 8 weeks after administration of the last induction dose. Preferably, the first maintenance dose is administered 4 weeks after the last induction dose of the induction treatment phase.

In a specific embodiment, the maintenance dose in the method is 100-1000 mg, e.g., 100, 200, 300, 400, 500, 600, 700, 800, 900, or 1000 mg. It will be appreciated that the maintenance dose may be 100, 200, 300, 400, 500, 600, 700, 800, 900 or 1000 mg or a range consisting of any two thereof, for example, 200-900 mg, 200-800 mg, 200-600 mg, etc. In particular, the maintenance dose is 200 mg.

In a specific embodiment, the method comprises administering to a patient in need an anti-IL-23p19 antibody, wherein the administration regimen comprises:
a) an induction treatment phase: administering to a patient in need thereof 3 induction doses of an anti-IL-23p19 antibody by intravenous infusion at 4-week intervals, each induction dose being 100-1000 mg;
b) a maintenance treatment phase: administering to the patient maintenance doses of the anti-IL-23p19 antibody by subcutaneous injection at intervals of 4-8 weeks, the first maintenance dose being administered 2-8 weeks after the last induction dose, each maintenance dose being 100-1000 mg.

In a specific embodiment, the method comprises administering to a patient in need an anti-IL-23p19 antibody, wherein the administration regimen comprises:
a) an induction treatment phase: administering to a patient in need thereof 3 induction doses of an anti-IL-23p19 antibody by intravenous infusion at 4-week intervals, each induction dose being 200-600 mg;
b) a maintenance treatment phase: administering to the patient maintenance doses of the anti-IL-23p19 antibody by subcutaneous injection at intervals of 4-8 weeks, the first maintenance dose being administered 2-8 weeks after the last induction dose, each maintenance dose being 200-600 mg.

In a specific embodiment, the method comprises administering to a patient in need an anti-IL-23p19 antibody, wherein the administration regimen comprises:
a) an induction treatment phase: administering to a patient in need thereof 3 induction doses of the anti-IL-23p19 antibody by intravenous infusion at 4-week intervals, each induction dose being 200-600 mg;
b) a maintenance treatment phase: administering to the patient maintenance doses of the anti-IL-23p19 antibody by subcutaneous injection at an interval of 4 weeks or 8 weeks, the first maintenance dose being administered 2-8 weeks after the last induction dose, each maintenance dose being 200-600 mg.

In a specific embodiment, the method comprises administering to a patient in need an anti-IL-23p19 antibody, wherein the administration regimen comprises:
a) induction treatment phase: administering to a patient in need thereof 3 induction doses of the anti-IL-23p19 antibody by intravenous infusion at an interval of 4 weeks, each induction dose being 200 mg or 600 mg;
b) a maintenance treatment phase: administering to the patient maintenance doses of the anti-IL-23p19 antibody by subcutaneous injection at an interval of 4 weeks or 8 weeks, the first maintenance dose being administered 2-8 weeks after the last induction dose, each maintenance dose being 200 mg.

In a specific embodiment, the method comprises administering to a patient in need an anti-IL-23p19 antibody, wherein the administration regimen comprises:
a) an induction treatment phase: administering to a patient in need thereof 3 induction doses of the anti-IL-23p19 antibody by intravenous infusion at 4-week intervals, each induction dose being 200 mg or 600 mg;
b) a maintenance treatment phase: administering to the patient maintenance doses of the anti-IL-23p19 antibody by subcutaneous injection at an interval of 4 weeks or 8 weeks, the first maintenance dose being administered 4 weeks after the last induction dose, each maintenance dose being 200 mg .

In a specific embodiment, the method comprises administering to a patient in need an anti-IL-23p19 antibody, wherein the administration regimen comprises:
a) an induction treatment phase: administering to a patient in need thereof 3 induction doses of the anti-IL-23p 19 antibody by intravenous infusion at 4-week intervals, each induction dose being 200 mg or 600 mg;
b) a maintenance treatment phase: administering maintenance doses of the anti-IL-23p19 antibody to the patient by subcutaneous injection at an interval of 4 weeks or 8 weeks, the first maintenance dose being administered 2-8 weeks after the last induction dose, each maintenance dose being 200 mg, the administration continuing until Week 44.

In a specific embodiment, the method comprises administering to a patient in need an anti-IL-23p19 antibody, wherein the administration regimen comprises:
a) an induction treatment phase: administering to a patient in need thereof 3 induction doses of the anti-IL-23p19 antibody by intravenous infusion at 4-week intervals, each induction dose being 200 mg or 600 mg;
b) a maintenance treatment phase: administering maintenance doses of the anti-IL-23p19 antibody to the patient by subcutaneous injection at an interval of 4 weeks or 8 weeks, the first maintenance dose being administered 4 weeks after the last induction dose is administered, each maintenance dose being 200 mg, the administration continuing until Week 44.

In a specific embodiment, the method further comprises:
a) at the end of the induction treatment phase, the Mayo score is assessed at Week 11-12,
b) during the maintenance treatment phase, subjects who achieved a clinical response at weeks 12-13 are re-randomized in a 1:1 ratio to be administrated the anti-IL-23p19 antibody 200 mg by subcutaneous injection at an interval of 4 weeks or the anti-IL-23p19 antibody 200 mg by subcutaneous at an interval of 8 weeks until Week 44, with safety follow-up until Week 52.

In a specific embodiment, the method further comprises
a) at the end of the induction treatment phase, the Mayo score is assessed at Week 12 to evaluate the efficacy response of the subject to the study drug;
b) at the start of the maintenance treatment phase, subjects who achieved a clinical response at Week 12 are re-randomized in a 1:1 ratio to be administrated 200 mg by subcutaneous at an interval of 4 weeks or the anti-IL-23p19 antibody 200 mg by subcutaneous injection at an interval of 8 weeks until Week 4 4, with safety follow-up until Week 52.

In one embodiment, the administration regimen of the present invention further comprises an extended induction phase (or an extended induction treatment phase) performed after the induction treatment phase and before the maintenance treatment phase described herein. In one embodiment, the patient does not achieve a clinical response 2-8 weeks after receiving the last induction dose of the induction treatment phase and thus receives an extended induction phase.

In one embodiment, the administration regimen of the invention comprises an extended induction phase that is conducted after the induction phase and before the maintenance phase:
a1) an extended induction phase: administering the patient an extended induction dose of the anti-IL-23p19 antibody one or more times, wherein the extended induction dose is 100-1000 mg; preferably, the patient enters a maintenance treatment phase once the administration of the extended induction dose has produced a clinical response.

In one embodiment, the extended induction dose is administered by intravenous injection. More preferably, the extended induction dose is administered by intravenous infusion.

In one embodiment, the first extended induction dose is administered 2-8 weeks after the last induction dose of the induction treatment phase, preferably 2, 3, 4, 5, 6, 7 or 8 weeks after the last induction dose, more preferably 4 weeks after the last induction dose.

In one embodiment, the extended induction dose may be administered one or more times, e.g., one, two, three, or four times. Preferably, the extended induction dose is administered 1-3 times. More preferably, the extended induction dose is administered 3 times. It will be appreciated that each of the extended induction doses may or may not be equal. In some embodiments, when multiple extended induction doses are administered, the extended induction doses are administered at 4 week intervals.

In one embodiment, the patient may receive a maintenance treatment phase once the administration of the extended induction dose has produced a clinical response.

In one embodiment, the patient achieves clinical response 2-8 weeks after receiving the last extended induction dose of the extended induction phase and then the maintenance phase.

In a specific embodiment, the administration regimen in the method further comprises an extended induction treatment phase: after 3 induction doses of the anti-IL-23p19 antibody are administered to the patient in need thereof at 4-week intervals, if the patient does not achieve a clinical response 2-8 weeks after the last induction dose is administered, 3 extended induction doses are administered to the patient, each extended induction dose being 100-1000 mg; if the patient produces a clinical response to the administration of the extended induction dose, the maintenance treatment phase is entered.

In a specific embodiment, the administration regimen in the method further comprises an extended induction treatment phase: after 3 induction doses of the anti-IL-23p19 antibody are administered at 4-week intervals to the patient in need, if the patient does not achieve a clinical response 4 weeks after the last induction dose is administered, 3 extended induction doses are administered to the patient, each extended induction dose being 100-1000 mg; if the patient produces a clinical response to the administration of the extended induction dose, the maintenance treatment phase is entered.

In a specific embodiment, each extended induction dose in the method is 100 mg, 200 mg, 300 mg, 400 mg, 500 mg, 600 mg, 700 mg, 800 mg, 900 mg, or 1000 mg. It will be appreciated that the extended induction dose may be 100 mg, 200 mg, 300 mg, 400 mg, 500 mg, 600 mg, 700 mg, 800 mg, 900 mg or 1000 mg, or a range consisting of any two thereof, for example, 200-900 mg, 200-800 mg, 200-600 mg, etc. In particular, the extended induction dose is 600 mg.

In some embodiments, the administration regimen of the present invention further comprises a maintenance treatment phase following the extended induction phase described herein. In particular, the maintenance treatment phase is substantially the same as the maintenance treatment phase described herein that occurs after the induction treatment phase.

In some embodiments, the administration regimen of the present invention comprises:
a) an induction treatment phase: administering one or more induction doses of the anti-IL-23p19 antibody to a patient in need thereof, wherein each the induction doses is 100-1000 mg;
a1) an extended induction phase: administering the patient an extended induction dose of the anti-IL-23p19 antibody one or more times, wherein the extended induction dose is 100-1000 mg; preferably, the patient enters a maintenance treatment phase once the administration of the extended induction dose has produced a clinical response; and
b) a maintenance treatment phase: administering to the patient one or more maintenance doses of the anti-IL-23p19 antibody, the first maintenance dose being administered 2-8 weeks after the last induction dose is administered, wherein each maintenance doses is 100-1000 mg.

In some embodiments, the maintenance treatment phase comprises administering to the patient one or more maintenance doses of the anti-IL-23p19 antibody, wherein the maintenance dose is 100-1000 mg. Preferably, the maintenance dose is administered by subcutaneous injection.

In a specific embodiment, the maintenance dose may be administered one or more times. It will be appreciated that the maintenance doses may each be equal or unequal. When multiple maintenance doses are administered, the maintenance doses are administered at intervals of 2-8 weeks, e.g., 2, 3, 4, 5, 6, 7 or 8 weeks. In particular, the maintenance doses are administered at an interval of 4 weeks or 8 weeks. In particular, the maintenance treatment phase lasts for 32 weeks from the administration of the first maintenance dose.

In a specific embodiment, the administration regimen in the method further comprises, after the extended induction phase, administering the first maintenance dose 2-8 weeks, e.g., 2 weeks, 3 weeks, 4 weeks, 5 weeks, 6 weeks, 7 weeks, or 8 weeks after administering the last extended induction dose. Preferably, the first maintenance dose is administered 4 weeks after the last extended induction dose of the extended induction phase.

In one specific embodiment, in the method, a maintenance dose of the anti-IL-23p19 antibody is administered to a patient in need thereof by subcutaneous injection at 4-week intervals after an extended induction phase.

In a specific embodiment, the method further comprises administering a maintenance dose of 100 mg, 200 mg, 300 mg, 400 mg, 500 mg, 600 mg, 700 mg, 800 mg, 900 mg, or 1000 mg after the extended induction phase. It will be understood that the maintenance dose is 100 mg, 200 mg, 300 mg, 400 mg, 500 mg, 600 mg, 700 mg, 800 mg, 900 mg, or 1000 mg, or a range consisting of any two thereof, such as 200-900 mg, 200-800 mg, or 200-600 mg. In particular, the maintenance dose is 200 mg.

In a specific embodiment, the administration regimen in the method further comprises that the subject who fails to achieve a clinical response at Week 12 may choose to enter an extended induction phase and receive 600 mg of the anti-IL-23p19 antibody by intravenous infusion at Weeks 12, 16, and 20.

In one specific embodiment, after the induction dose is extended, the subject that produces a clinical response enters the maintenance treatment phase, and the first maintenance dose is administered 4 weeks after the last extended induction dose, with 200 mg of the anti-IL-23p19 antibody by subcutaneous injection until Week 56.

In a specific embodiment, the method comprises administering to a patient in need thereof an anti-IL-23p19 antibody, wherein the administration regimen comprises:
a) an induction treatment phase: administering to a patient in need thereof 3 induction doses of the anti-IL-23p19 antibody by intravenous infusion at 4-week intervals, each induction dose being 200 mg or 600 mg;
b) a maintenance treatment phase: administering to the patient maintenance doses of the anti-IL-23p19 antibody by subcutaneous injection at an interval of 4 weeks or 8 weeks, the first maintenance dose being administered 4 weeks after the last induction dose, each maintenance dose being 200 mg, wherein the amino acid sequence of the heavy chain CDR1 of the antibody comprises or consists of SEQ ID NO. 1, the amino acid sequence of the heavy chain CDR2 comprises or consists of SEQ ID NO. 2, and the amino acid sequence of the heavy chain CDR3 comprises or consists of SEQ ID NO. 3; the amino acid sequence of the light chain CDR1 of the antibody comprises or consists of SEQ ID NO. 4, the amino acid sequence of the light chain CDR2 comprises or consists of SEQ ID NO. 5, and the amino acid sequence of the light chain CDR3 comprises or consists of SEQ ID NO. 6, and the boundaries of the CDRs are determined by AbM scheme.

In one specific embodiment, the administration regimen in the method further comprises an extended induction treatment phase: administering to a patient in need thereof 3 induction doses of the anti-IL-23p19 antibody at 4-week intervals, and if the patient does not achieve a clinical response 4 weeks after the last induction dose is administered, 3 extended induction doses are administrated to the patient, each extended induction dose being 600 mg; if the patient produces a clinical response to the administration of the extended induction dose, a maintenance treatment phase is entered, wherein the first maintenance dose is administered 4 weeks after the last extended induction dose is administered, with 200 mg of the anti-IL-23p19 antibody by subcutaneous injection until Week 56, wherein the amino acid sequence of the heavy chain CDR1 of the antibody comprises or consists of SEQ ID NO. 1, the amino acid sequence of the heavy chain CDR2 comprises or consists of SEQ ID NO. 2, and the amino acid sequence of the heavy chain CDR3 comprises or consists of SEQ ID NO. 3; the amino acid sequence of the light chain CDR1 of the antibody comprises or consists of SEQ ID NO. 4, the amino acid sequence of the light chain CDR2 comprises or consists of SEQ ID NO. 5, and the amino acid sequence of the light chain CDR3 comprises or consists of SEQ ID NO. 6, and the boundaries of the CDRs are determined by the AbM scheme.

In one embodiment, the administration regimen of the present invention comprises:
a) an induction treatment phase: administering to a patient in need thereof 3 induction doses of the anti-IL-23p19 antibody by intravenous infusion at 4-week intervals, each induction dose being 200 mg or 600 mg; and
a1) an extended induction phase: administering to a patient in need thereof 3 extended induction doses of the anti-IL-23p19 antibody by intravenous infusion at 4-week intervals, each extended induction dose being 600 mg; and
b) a maintenance treatment phase: administering to the patient maintenance doses of the anti-IL-23p19 antibody by subcutaneous injection at an interval of 4 weeks or 8 weeks, the first maintenance dose being administered 4 weeks after the last extended induction dose is administered, each maintenance dose being 200 mg, the administration continuing until Week 56.

In a specific embodiment, the method comprises administering to a patient in need thereof an anti-IL-23p19 antibody, wherein the administration regimen comprises:
a) an induction treatment phase: administering to a patient in need thereof 3 induction doses of an anti-IL-23p19 antibody by intravenous infusion at an interval of 4 weeks, each induction dose being 200 mg or 600 mg; performing Mayo scoring at week 12 to evaluate the efficacy response of the anti-IL-23p19 antibody in the subject.
b) a maintenance phase: subjects who achieve a clinical response at Week 12 are re-randomized to be administrated maintenance doses of the anti-IL-23p19 antibody by subcutaneous injection at 4- or 8-week intervals, each maintenance dose being 200 mg, the administration continuing until Week 44, with safety follow-up until Week 52.
c) an extended induction phase: subjects who fail to achieve a clinical response at Week 12 may choose to enter the clinical induction phase and are administrated 600 mg of the anti-IL-23p19 antibody at Weeks 12, 16, and 20 by intravenous infusion. The Mayo score is performed at Week 24 to evaluate the efficacy response of the anti-IL-23p19 antibody in the subjects.
d) a maintenance phase: subjects who achieved clinical response in the extended induction phase at Week 24 entered the maintenance phase by subcutaneous injection of 200 mg of anti-IL-23p19 antibody at 4-week intervals until Week 56, with safety follow-up until Week 64, while subjects who did not achieve a clinical response at Week 24 discontinued from the study.

In one specific embodiment, route of administration is subcutaneous injection or intravenous infusion.

In one specific embodiment, the subject must have previously received at least one biologics treatment or be the first time to use biologics: a. having previously received at least one biologics treatment refers to having received an anti-tumor necrosis factor-α (TNF-α) antagonist such as adalimumab, infliximab, etc., an anti-integrin antibody such as vedolizumab, or other experimental biological treatments, and the patient has poor response or loss of response or intolerance to the above treatments; b. Subjects who are the first time to use biologics must meet at least one of the following criteria:
- Poor response or loss of response or intolerance to treatment with oral 5-aminosalicylic acid;
- Poor response or intolerance to treatment with oral glucocorticoids;
- Poor response or intolerance to treatment with immunomodulators (6-mercaptopurine or azathioprine);
- Poor response or intolerance to JAK (Janus Kinases) inhibitor treatment /experimental treatment.

In one embodiment, the heavy chain of the IL-23p19 antibody of the present invention contains an N-glycosylation site located at asparagine 295 of the heavy chain. Thus, in one embodiment, the anti-IL-23p19 antibody of the present invention is a glycosylated antibody, e.g., an antibody glycosylated with asparagine at position 295.

In one specific embodiment, the anti-IL-23p19 antibody comprises a heavy chain variable region VH and a light chain variable region VL, wherein the heavy chain variable region comprises or consists of SEQ ID NO:7 or a sequence having at least 90%, 95%, 98% or 99% identity thereto, and the light chain variable region comprises or consists of SEQ ID NO:8 or a sequence having at least 90%, 95%, 98% or 99% identity thereto:
Sequence (SEQ ID NO: 7) 
QVQLVQSGAEVKKPGASVKVSCKASGYTFTSYLMHWVRQAPGQGLEWMGYINPYNEGT
NYAQKFQGRVTMTRDTSISTAYMELSRLRSDDTAVYYCARNWDLPYWGQGTLVTVSS;
Sequence (SEQ ID NO: 8)

DIQMTQSPSSLSASVGDRVTITCRASQSISDYLHWYQQKPGKAPKLLIKYASQSMSGVPSRF
SGSGSGSDFTLTISSLQPEDFATYYCQQGHSFPFTFGQGTKLEIK.

In one embodiment, the IL-23p19 antibody of the present invention comprises a heavy chain and/or a light chain, wherein the heavy chain comprises or consists of a sequence set forth in SEQ ID NO: 9 or a sequence having at least 90%, 95%, 98% or 99% identity thereto, and the light chain comprises or consists of a sequence set forth in SEQ ID NO: 10 or a sequence having at least 90%, 95%, 98% or 99% identity thereto. In a specific embodiment, the IL-23p19 antibody is an IgG1 antibody comprising a heavy chain and a light chain, wherein the heavy chain comprises or consists of a sequence of SEQ ID NO: 9 or a sequence having at least 90%, 95%, 98% or 99% identity thereto, and wherein the light chain comprises or consists of a sequence of SEQ ID NO: 10 or a sequence having at least 90%, 95%, 98% or 99% identity thereto. In one embodiment, the IL-23p19 antibody of the present invention comprises or consists of two heavy chains and two light chains, e.g., two identical heavy chains and two identical light chains.

In one embodiment, the IL-23p19 antibody of the present invention comprises a heavy chain set forth in SEQ ID NO: 9 and a light chain set forth in SEQ ID NO: 10; for example, the IL-23p19 antibody comprises or consists of 2 heavy chains set forth in SEQ ID NO: 9 and 2 light chains set forth in SEQ ID NO: 10.
Sequence (SEQ ID NO: 9) 
QVQLVQSGAEVKKPGASVKVSCKASGYTFTSYLMHWVRQAPGQGLEWMGYINPYNEGT
NYAQKFQGRVTMTRDTSISTAYMELSRLRSDDTAVYYCARNWDLPYWGQGTLVTVSSAS
TKGPSVFPLAPSSKSTSGGTAALGCLVKDYFPEPVTVSWNSGALTSGVHTFPAVLQSSGLY
SLSSVVTVPSSSLGTQTYICNVNHKPSNTKVDKKVEPKSCDKTHTCPPCPAPELLGGPSVFL
FPPKPKDTLYITREPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSRDELTKNQV
SLTCLVKGFYPSDIAVEWESNGQPENNYKTTPPVLDSDGSFFLYSKLTVDKSRWQQGNVFS
CSVMHEALHNHYTQKSLSLSPG
Sequence (SEQ ID NO: 10)

DIQMTQSPSSLSASVGDRVTITCRASQSISDYLHWYQQKPGKAPKLLIKYASQSMSGVPSRF
SGSGSGSDFTLTISSLQPEDFATYYCQQGHSFPFTFGQGTKLEIKRTVAAPSVFIFPPSDEQLK
SGTASVVCLLNNFYPREAKVQWKVDNALQSGNSQESVTEQDSKDSTYSLSSTLTLSKADY
EKHKVYACEVTHQGLSSPVTKSFNRGEC

Preferably, the IL-23p19 antibody is the anti-IL-23p19 antibody 17D1-YTE disclosed in PCT Application No. PCT/CN2019/121261 (International Application Date: Nov. 27, 2019, International Publication No. WO2020/108530A1), which consists of SEQ ID NO:9 and SEQ ID NO:10. This patent application is incorporated herein in its entirety as if it were set forth in its entirety herein.

In one specific embodiment, the anti-IL-23p19 antibody comprises a heavy chain sequence set forth in SEQ ID NO: 9 and a heavy chain amino acid sequence set forth in SEQ ID NO:10.

In one embodiment, the IL-23p19 antibody of the present invention is a full-length antibody. In one embodiment, the IL-23p19 antibody of the present invention also encompasses antigen-binding fragments.

In one specific embodiment, the anti-IL-23p19 antibody is recombinantly expressed in HEK 293 cells or CHO cells.

The IL-23p19 antibody of the present invention may be formulated into a formulation for administration. For example, the formulation formula of the anti-IL-23p19 antibody of the present invention is as follows: 100.0 mg/mL anti-IL23p19 subunit antibody, 0.76 mg/mL histidine, 1.08 mg/mL histidine hydrochloride, 50.00 mg/mL sorbitol, and 0.5 mg/mL polysorbate 80, pH 6.0. Preferably, the formulation formula of the IL-23p19 antibody can be prepared according to the method for the formulation comprising the anti-IL-23p19 antibody, the preparation method therefor, and the use thereof disclosed in PCT Application No. PCT/CN2021/093219 (International Application Date: May 12, 2021, International Publication No. WO2021/228113A1). This patent application is incorporated herein in its entirety as if it were set forth in its entirety herein.

### Single Dosage unit

A single dosage unit, comprising: an effective amount of the anti-IL-23p19 antibody of the present invention, preferably comprising a fixed dose of 100-1000 mg. The single-dosage unit is used for administration according to the administration regimen of the present invention to prevent or treat inflammatory bowel disease, e.g., ulcerative colitis or Crohn's disease.

The unit dosage forms described herein are preferably for parenteral administration, e.g., for injection, e.g., intravenous injection (including intravenous infusion) or subcutaneous injection. The unit dosage form may be a vial, an ampoule, or a pre-filled syringe for injection, containing a solution or a lyophilized powder of the IL-23p19 antibody. More preferably, the unit dosage form described herein is a pre-filled syringe comprising a solution or lyophilized powder of the IL-23p19 antibody for parenteral administration.

In one embodiment, the IL-23p19 antibody is presented in a pre-filled syringe, and preferably, the pre-filled syringe comprises a 200 mg/mL or 600 mg/mL IL-23p19 antibody solution.

### Kit of parts

A kit of parts comprising an effective amount of the anti-IL-23p19 antibody of the present invention, and in particular, a package insert printed with instructions for using the anti-IL-23p19 antibody to prevent or treat ulcerative colitis in an individual.

### Use

Use of the anti-IL-23p19 antibody, the single-dosage unit, or the kit of parts of the present invention in the manufacture of a medicament for preventing or treating inflammatory bowel disease, such as ulcerative colitis or Crohn's disease or colitis (especially moderate to severe ulcerative colitis).

It is to be understood that the terminology used herein is for the purpose of describing particular embodiments only, and is not intended to be limiting of the invention. The scope of the present invention is defined solely by the appended claims.

Unless defined otherwise, all technical and scientific terms used herein have the same meaning as commonly understood by one of ordinary skill in the art to which this invention belongs. Herein, technical and scientific terms that are not specifically defined have the meanings that are commonly understood by those skilled in the art to which the present invention pertains.

Any of the embodiments, technical features, and definitions described herein may be combined arbitrarily to form embodiments that are not directly described in the specification but comply with the purpose of the present invention, and these embodiments are also included in the scope of the present invention.

The administration regimen described herein (including the active agent IL-23p19 antibody, the dose of administration, the mode of administration, the administration interval, the number of administrations, the duration of administration, or the like) and any one of the features thereof can be applied to any one of the aspects of the present invention, such as the first to eighth aspects described above. Moreover, any of the embodiments, technical features, definitions, and any combination thereof described herein for one aspect may also be applied to other aspects, such as the first to eighth aspects described above. For example, any of the embodiments, technical features, definitions, and any combination thereof described herein for the first aspect or the second aspect may be equally applied to the third to eighth aspects as described above.

### EXAMPLES

The following examples are provided to illustrate the invention. These examples are merely illustrative and do not in any way limit the scope of the invention and the appended claims. Likewise, the invention is not limited to any particular preferred embodiment described herein. Variations may be made by those skilled in the art without departing from the spirit and scope of the invention.

### Example 1. Preparation and purification of an IL-23p19 antibody

The antibody 17D1-YTE that specifically binds to IL-23p19 was obtained as described in PCT Application No. PCT/CN2019/121261. The antibody has the amino acid sequence of SEQ ID NO:9 and SEQ ID NO:10. PCT Application No. PCT/CN2019/121261 is incorporated herein by reference in its entirety.

Briefly, the antibody was recombinantly expressed in CHO cells, and the IL-23p19 antibody sample used in the present invention was obtained by purification using an affinity chromatography method and purified using a cation exchange chromatography method.

### Example 2. Preparation of formulation formula

### 2.1 Experimental procedures

The formulas of the antibody formulations of the present invention were investigated as described in PCT Application No. PCT/CN2021/093219. Specifically, 20 mM sodium citrate and 150 mM sodium chloride were prepared, and the pH was adjusted with hydrochloric acid. The purified IL-23p19 antibody in Example 1 was exchanged into the above buffers with different pH values by ultrafiltration, and the protein content was adjusted to 50 mg/mL. The solutions were filtered and aliquoted into vials, followed by plugging and capping.

The determined injection formulation formula of antibody: 100 mg/mL recombinant anti-interleukin 23p19 subunit antibody, 0.76 mg/mL histidine, 1.08 mg/mL histidine hydrochloride, 50.00 mg/mL sorbitol, 0.50 mg/mL polysorbate 80, pH 6.0 ± 0.3. According to the formula, the antibody injection formulation of the present invention was prepared at a strength of 100 mg (1 mL)/vial, and was packaged in a vial. The product is a clear to slightly opalescent, colorless to yellowish liquid, free of particles. The injection formulation was used in Example 3.

Corresponding placebo: injection solution, strength: 1 mL/vial. The excipients include: 0.76 mg/mL histidine, 1.08 mg/mL histidine hydrochloride, 50.00 mg/mL sorbitol, and 0.5 mg/mL polysorbate 80, pH 6.0. The product is packaged in a vial, clear to slightly opalescent, colorless to yellowish liquid, free of particles. This injection formulation was used as placebo in Example 3.

### Example 3. Clinical Studies

The anti-IL-23p19 antibody of the present invention showed good safety and tolerability in a phase I clinical study in healthy volunteers. No Grade ≥ 3 treatment-emergent adverse event (TRAE) occurred. TRAEs with an incidence of ≥ 5% were injection site erythema (20.0%), upper respiratory tract infection (14.3%), and rhinorrhea (11.1%), which were basically consistent with the existing study results of drugs with the same target, and no new safety risks were found. The above results support further clinical efficacy and safety studies of the anti-IL-23p19 antibody.

### 3.1 Study Objectives

Primary Objective:
- To evaluate the efficacy of the anti-IL-23p19 antibody in inducing therapy achieving a clinical remission in patients with moderate to severe active ulcerative colitis (UC).

Secondary objective:
- To evaluate the efficacy of the anti-IL-23p19 antibody in induction therapy in achieving clinical response, symptomatic remission, endoscopic remission and mucosal healing in patients with moderately to severely active ulcerative colitis;
- To evaluate the effectiveness of the anti-IL-23p19 antibody in maintenance therapy in achieving clinical remission, clinical response, symptomatic remission, endoscopic remission and mucosal healing in patients with moderately to severely active ulcerative colitis;
- To evaluate the effect of induction therapy and maintenance therapy with the anti-IL-23p19 antibody on health-related quality of life in patients with moderately to severely active ulcerative colitis;
- To evaluate the safety of the anti-IL-23p19 antibody in induction therapy and maintenance therapy in patients with moderate to severe active ulcerative colitis;
- To evaluate the pharmacokinetic characteristics of the anti-IL-23p19 antibodies in patients with moderate to severe active ulcerative colitis;
- To evaluate the immunogenicity of the anti-IL-23p19 antibodies in patients with moderately to severely active ulcerative colitis.

### 3.2 Study Design Rationale

### 3.2.1. Rationale for dose selection

Based on the characteristics of the disease indicated for UC and the mechanism of action of the IL-23 molecular pathway, combined with clinical safety, efficacy, and operational convenience, it is proposed to recommend a phase II study with an intravenous infusion dose of 200 mg Q4W and 600 mg Q4W in the induction treatment phase and a subcutaneous injection dose of 200 mg Q4W and 200 mg Q8W in the maintenance treatment phase.

### 3.3 Study Design

This study is a multicenter, randomized, double-blind, placebo-controlled phase II clinical study to evaluate the efficacy and safety of the anti-IL-23p19 antibody in the induction and maintenance treatment of patients with moderately to severely active ulcerative colitis (modified Mayo score 4-9, with endoscopic score ≥ 2). Approximately 150 subjects will be randomized, of which approximately 30%-50% must have received at least 1 biologics therapy, and approximately 50%-70% must be the first time to use biologics. After no more than 6 weeks of screening, patients with moderately to severely active ulcerative colitis who meet the enrollment criteria of the study will be randomly assigned to 1 of the 3 induction treatment groups in a 1:1:1 ratio, with previous treatment with biologics used as a stratification factor.
- Induction treatment phase: The 12-week induction treatment phase was used to evaluate the efficacy and safety of intravenous infusion of the anti-IL-23p19 antibody in the induction treatment of target patients. Subjects were randomly assigned to be administrated placebo, 200 mg of the anti-IL-23p19 antibody, or 600 mg of the anti-IL-23p19 antibody by intravenous infusion at weeks 0, 4, and 8. Subjects were evaluated for efficacy response to the study drug at Week 12.
- Maintenance treatment phase: This phase was intended to evaluate the efficacy and safety of subcutaneous injection of the anti-IL-23p19 antibody in maintenance treatment of target patients. Subjects who achieved a clinical response at Week 12 continued to participate in the maintenance treatment phase. Subjects who received placebo in the induction treatment phase continued to be administrated placebo by subcutaneous injection every 4 weeks in the maintenance treatment phase; Subjects who received the anti-IL-23p19 antibody in the induction treatment phase were re-randomized in a 1:1 ratio to be administrated the anti-IL-23p19 antibody 200 mg by subcutaneous injection at an interval of 4 weeks, or the anti-IL-23p19 antibody 200 mg by subcutaneous injection at an interval of 8 weeks until Week 44, and with safety follow-up until Week 52. For the subjects receiving anti-IL-23p19 antibody 200 mg by subcutaneous injection an interval of 8 weeks in the maintenance treatment phase, placebo was injected subcutaneously at weeks 16, 24, 32, and 40 to maintain the blind.
- Expansion treatment phase: Subjects who failed to achieve a clinical response at Week 12 may choose to enter the expansion phase. All subjects who enter this phase, regardless of whether placebo or the anti-IL-23p19 antibody is used in the induction phase, received the anti-IL-23p19 antibody 600 mg by intravenous infusion at start of this phase (Week 12), Week 16, and Week 20. The efficacy response of the anti-IL-23p19 antibody was evaluated in these subjects at Week 24. Subjects who achieve a clinical response started to be administrated subcutaneous injection of the anti-IL-23p19 antibody 200 mg every 4 weeks until Week 56 and with safety follow-up until Week 64. Subjects who failed to achieve a clinical response were discontinued from the study.

Subjects and investigators remained blinded in the induction treatment phase and maintenance treatment phase until the end of the study; the expansion phase is an open study period, in which blinding is not performed. The antibody injection formulations and placebo prepared as in Example 2 were used in this study.

### 3.4 Study Population

### Inclusion Criteria

Potential subjects must meet all of the following criteria to be enrolled in this study.
(1) Males or females aged 18 to 75 years;
(2) Diagnosis of ulcerative colitis for at least 3 months, including endoscopic evidence to support UC and histopathological evidence to support the diagnosis of UC;
(3) Moderately severe ulcerative colitis, defined as a modified Mayo score ≥ 4 points and an endoscopic score ≥ 2 points;
(4) Subjects must have received at least one biologics or be the first time to use biologics:
   4a. Previous treatment with at least one biologics refers to treatment with an anti-tumor necrosis factor-α (TNF-α) antagonist such as adalimumab, infliximab, etc., an anti-integrin antibody such as vedolizumab, or other experimental biological therapies, and the patient has poor response or loss of response or intolerance to the above treatments;
      - Poor response: Patients who still have persistent symptoms or signs of active disease despite induction therapy using the induction administration regimen recommended in the package insert (induction therapy using the recommended trial dose for the experimental treatment);
         - Loss of response: Symptoms or signs of active disease appear during the maintenance administration after previous clinical benefit is achieved;
      - Intolerance: dose reduction or termination of treatment due to adverse reactions related to the above formulations.
   4b. Subjects who are the first time to use biologics must meet at least one of the following criteria:
      - Poor response, loss of response, or intolerance to treatment with oral 5-aminosalicylic acid; defined as persistent symptoms or signs of active disease despite induction therapy at a dose of at least 2 g/day (or equivalent dose) of mesalamine for at least 2 weeks, or symptoms or signs of active disease during maintenance therapy phase, or intolerance due to adverse drug reactions.
      - Poor response or intolerance to treatment with oral glucocorticoids; defined as persistent symptoms or signs of active disease despite treatment with oral prednisone (or equivalent dose) at a dose of at least 30 mg/day for at least 2 weeks; or intolerance due to glucocorticoid-related adverse reactions.
      - Hormone therapy dependence; defined as the inability to reduce the dose of glucocorticoids to less than 10 mg/day of prednisone (or equivalent dose) within 3 months after the start of glucocorticoid therapy without showing symptoms or signs of active disease; or relapse within 3 months after completing a course of glucocorticoid therapy.
      - Poor response or intolerance to treatment with the immunomodulator (6-mercaptopurine or azathioprine); defined as persistent symptoms or signs of active disease despite treatment with 6-mercaptopurine at least 0.75 mg/kg/day or azathioprine at least 1 mg/kg/day for at least 3 months; or intolerance due to a drug-related adverse reaction.
      - Poor response or intolerance to JAK (Janus Kinases) inhibitor treatment /experimental treatment. Defined as persistent symptoms or signs of active disease that persist for at least 2 months at the labeled or test dose of the JAK inhibitor; or intolerable due to drug-related adverse reactions.
   (5) Prior to the first time to use the study drug, UC-related therapeutic drugs must meet the following requirements:
      - discontinuation of TNF-α antagonist for at least 8 weeks;
      - discontinuation of the anti-integrin antibody vedolizumab for at least 12 weeks;
      - discontinuation of other experimental biologics for at least 8 weeks;
      - at least 2 weeks stable at a prescribed dose if taking oral aminosalicylic acid preparations (sulfasalazine or other 5- aminosalicylic acid); having been at least 2 weeks if discontinued;
      - at least 2 weeks stable at a prescribed dose if oral glucocorticoids are taken; having been at least 2 weeks if discontinuation;
      - if receiving traditional immunomodulators (6-mercaptopurine or azathioprine or methotrexate), they should have been received for at least 12 weeks and stabilized at the prescribed dose for at least 4 weeks; having been at least 4 weeks if discontinuation;
      - discontinuation of cyclosporine, tacrolimus, pimecrolimus, 6-thioguanine, thalidomide, mycophenolate mofetil, and JAK inhibitor/experimental treatment for at least 4 weeks;
      - discontinuation of rectal glucocorticoids (injection into the rectum or sigmoid colon through foams, enemas, suppositories, etc.), and injection glucocorticoids for at least 2 weeks;
      - discontinuation of rectal aminosalicylic acid (administered as foams or enemas or suppositories into the rectum or sigmoid colon) for at least 2 weeks;
      - discontinuation of traditional Chinese medicine preparations (oral or enema) for at least 2 weeks;
      - discontinuation of parenteral nutrition for at least 2 weeks;
      - discontinuation of antibiotic therapy for the treatment of ulcerative colitis for at least 2 weeks;
      - discontinuation of selective leukocyte adsorption therapy for at least 2 weeks;
      - discontinuation of other experimental treatment for at least 4 weeks.
   (6) Fully understand the objectives of the trial, have a basic understanding of the pharmacological effects of the study drug and possible adverse reactions, and voluntarily sign the informed consent form in accordance with the spirit of the Declaration of Helsinki.

### Exclusion Criteria

Potential subjects to be enrolled in this study must not meet any of the following criteria.
(1) Previously treated with biologics targeting IL-12/23 and/or IL-23;
(2) Diagnosed wtih ischemic colitis, infectious colitis, radiation colitis, microscopic colitis, indeterminate colitis, etc., or suggestive of Crohn's disease;
(3) UC lesions are limited to the rectum or involve the colon < 15 cm;
(4) Evidence of toxic megacolon is found at screening;
(5) History or evidence of colonic atypical hyperplasia, adenomatous polypus (not resected prior to study entry), or gastrointestinal tumor;
(6) History of extensive colectomy, subtotal or total colectomy, ileostomy, or colostomy due to ulcerative colitis;
(7) Subjects who require surgery for ulcerative colitis or who are scheduled for elective surgery during the study period;
(8) Surgery for active gastrointestinal bleeding, peritonitis, intestinal obstruction, intestinal stenosis, abdominal abscess, or pancreatic abscess within 2 months prior to screening, or requiring surgery for the above conditions within 2 weeks prior to screening;
(9) Evidence showing subjects with serious, progressive, or uncontrollable cardiovascular, hematological, endocrine, renal, hepatic, respiratory, neurological, or psychiatric disorders;
(10) Severe opportunistic infections within 6 months prior to screening, such as severe or recurrent herpes zoster, active cytomegalovirus infection, pneumocystis carinii, histoplasma, aspergillus, and mycobacteria;
(11) A positive test for Clostridium difficile during the screening period or within 4 months prior to the first dose of medication, with exception of that repeated tests yield negative results and there are no persistent symptoms of infection by the pathogen (treatment and repeat testing may be performed during the screening period if time permits);
(12)Known history of recurrent or chronic infection, including but not limited to chronic renal infection, chronic chest infection, recurrent urinary tract infection, etc.;
(13)History of serious infection (e.g., sepsis, serious pneumonia, pyelonephritis, etc.), or hospitalization or intravenous antibiotic therapy for infection within 1 month prior to screening (except for non-serious upper respiratory tract infection, simple urinary tract infection, etc., as judged by the investigator as appropriate);
(14) Known or previous lymphoproliferative disorder, including lymphoma, or symptoms or signs of possible lymphoproliferative disorder within 5 years prior to screening, such as lymphadenopathy and/or splenomegaly;
(15)Known history of malignancy or malignancy (except for basal cell carcinoma, cutaneous squamous cell carcinoma, or cervical carcinoma in situ that have been successfully resected and treated within 5 years before screening without evidence of recurrence or metastasis);
(16) Evidence/symptoms of untreated latent or active tuberculosis, or a history of active tuberculosis at screening; except for those with no evidence/symptoms of active tuberculosis but positive QuantiFERON-TB [positive for T-SPOT in the absence of QuantiFERON-TB test] who have received appropriate anti-latent tuberculosis treatment prior to the first dose;
(17) meeting any one of the following laboratory test results at screening;
   - hemoglobin < 80 g/L; or any indicator of white blood cells, neutrophils, and platelets < lower limit of normal value (LLN), which is abnormal with clinical significance as judged by the investigator;
   - any of alanine aminotransferase (ALT), aspartate aminotransferase (AST), or total bilirubin (TBIL) > 2 times the upper limit of normal value (ULN);
   - Creatinine (Cr) > ULN;
(18) meeting any of the following virological examination results at screening :
   - positive for human immunodeficiency virus (HIV) antibody;
   - Hepatitis C virus (HCV) antibody positive with no history of successful treatment, wherein successful treatment is defined as negative HCV RNA after at least 24 weeks of antiviral treatment;
   - Hepatitis B virus (HBV) screening includes at least hepatitis B surface antigen (HbsAg), hepatitis B surface antibody (HbsAb), and hepatitis B core antibody (HbcAb), and the test result is: positive for hepatitis B surface antigen; or negative for HBsAg, if only HbcAb is positive, HBV DNA needs to be tested and the test result is positive;
(19) Participated in other clinical study within 4 weeks prior to screening and received the study drug or was within 5 half-lives of the study drug, or planned to participate in other clinical study during the study;
(20)Received BCG vaccination within 12 months prior to the first time to use the study drug, or planned to be administrated BCG vaccination during the study or within 12 months after the last study treatment;
(21) Received live or bacterial vaccines within 3 months prior to the first time to use the study drug, or plan to be administrated live or bacterial vaccines during the study or within 3 months after the last study treatment;
(22) Upon questioning, suspected or confirmed allergies or previous severe drug or food allergies, and/or allergies to the test drug or its ingredients;
(23) History of alcohol and drug abuse within 12 months prior to screening;
(24)Female subjects who are pregnant or breastfeeding, or of gestational age with a positive pregnancy test at screening and before administration;
(25) Those who plan to conceive during the study and within 6 months after the administration of study drug, or are unwilling to take appropriate contraceptive measures (such as condoms) as deemed by the physician during the study;
(26) Subjects who are deemed unsuitable for participation in this clinical trial by the investigator for various reasons.

### 3.5 Definition of End of Study

Completion of the last visit of the study will be considered as the completion of the study. Premature discontinuation of treatment/study for any reason prior to completion of the study is considered not to have completed the study. The end of the study refers to the last follow-up of the last subject.

### 3.5.1 Clinical Criteria for Discontinuation

This study may be temporarily suspended or prematurely terminated if there are sufficient reasonable reasons. The party suspending or terminating the study shall provide written notification to the subject, the investigator, the funding institution, and the regulatory authority and document the reason for suspension or termination of the study. If the study is prematurely terminated or suspended, the principal investigator should promptly notify the subjects and the Ethics Committee (EC) and provide the reasons for termination or suspension. When applicable, the investigator will contact the subject and notify the subject of the change in the scheduled visit time.

Circumstances that may require termination or suspension include but are not limited to:
- Determination of unexpected, significant, or unacceptable risks to subjects;
- The efficacy rationale should be discontinuation/interruption;
- Failure to meet protocol compliance requirements;
- Incomplete and/or insufficient data for evaluation;
- Plan to change or stop the development of the study drug.

The study may continue only if issues related to safety, protocol compliance, and data quality are addressed and EC and/or National Medical Products Administration requirements are met.

If the sponsor decides to discontinue providing the study drug, adequate notification will be provided to allow for appropriate adjustments to the subject's treatment.

### 3.6 Efficacy-Related Assessments

### 3.6.1 Mayo score

The "modified Mayo score" is derived from the Mayo scoring system for the severity and activity of ulcerative colitis. According to the recommendation of the U.S. Food and Drug Administration (FDA), physician's global assessment is not used. The modified Mayo score consists of three items, i.e., stool frequency, rectal bleeding, and endoscopic findings, with a score from 0 to 9.

Stool frequency and rectal bleeding data should be for 3 consecutive days within 1 week prior to the score visit, with the average of the 3-day period used as the score for stool frequency and rectal bleeding at the score visit. "Days" were excluded from the calculation if:
a. Use of drugs for constipation and diarrhea;
b. procedures or bowel preparation days that affect the stool frequency and/or rectal bleeding (e.g., enemas, liquid meals, and laxatives);
c. 2 days after the use of gastrointestinal motility inhibitors (e.g., diphenoxylate hydrochloride, atropine, loperamide, or other opioids);
d. 2 days after colonoscopy.

The "Symptomatic Mayo Score" is the modified Mayo score, without endoscopic findings, from 0 to 6 points.

The "Partial Mayo Score" is the Mayo score without endoscopic findings including stool, rectal bleeding, and physician's global assessment. It is only used to evaluate clinical recurrence and does not used as efficacy indicators .

"Clinical remission" was the primary endpoint of the study, defined as 0 point for rectal bleeding on the modified Mayo score, ≤1 point for stool frequency, and ≤1 point for endoscopic findings.

"Clinical response" is defined as a decrease from baseline in the modified Mayo score by ≥ 30% and ≥ 2 points, and a decrease from baseline in the rectal bleeding score of ≥ 1 points or a subscore of 0 or 1.

"Symptomatic remission" refers to a stool frequency score of 0 or 1 and a rectal bleeding score of 0.

"Endoscopic remission" refers to an endoscopic score of 0 or 1 in the modified Mayo score.An endoscopic score of 0 was considered complete endoscopic remission.

"Mucosal healing" refers to endoscopic remission with histopathologically centralized Geboes index (Geboes, K., Riddell, R., Ost, A., Jensfelt, B., Persson, T. & Lofberg, R. 2000. A reproducible grading scale for histological assessment of inflammation in ulcerative colitis. Gut, 47, 404-9).

### 3.6.2 Endoscopy/colon histopathology

Endoscopy was performed according to the study procedures visit, and biopsy samples were taken at the time of endoscopy from the site of UC inflammation. To ensure data quality and standardization to reduce evaluation bias, endoscopic scoring and histopathological reading scoring were evaluated by a central laboratory.

The video records of the complete endoscopic process and endoscopic images were transmitted to the central laboratory, and the central reading was performed by the trained digestive endoscopists of the central laboratory according to the reading charter.Endoscopic scores for modified Mayo score in clinical studies were obtained from a central reader.

Colon histopathological scoring was performed by the central laboratory. The biopsy samples were evaluated by trained pathologists of the central laboratory according to the reading charter, and the Geboes index was used for histopathological scoring of biopsy samples.

Two biopsy samples were be collected from a representative area 15-20 cm away from the anal verge. If this area is not a representative area of mucosal inflammation, biopsy samples can be collected from other representative areas. No more than 6 biopsy samples can be collected each time. Fresh biopsy samples were submitted to the central laboratory.

"Geboes index remission" means satisfying Grade 0 ≤ 0.3, Grade 1 ≤ 1.1, Grade 2A ≤ 2A.3, and Grade 2B, Grade 3, Grade 4, and Grade 5 all being 0 at the same time.

### 3.6.3 Inflammatory bowel disease quality of life questionnaire

The Inflammatory Bowel Disease Quality of Life Questionnaire (IBDQ) is a validated patient-reported outcome (PRO) instrument for the assessment of health-related quality of life in patients with inflammatory bowel disease, including ulcerative colitis (Guyatt. 1989).The questionnaire consists of 32 questions to evaluate 4 aspects of patients' life: intestinal symptoms, systemic symptoms, emotional capacity and social capacity. The score ranges from 32 to 224, the higher the score the better the quality of life.

### 3.6.4 Short Form Health Survey

The SF-36 scale, also known as the Medical Outcomes Study 36-Item Short Form Health Survey (SF-36), is a quality of life assessment tool that is completed by subjects and has been widely used to assess their health-related quality of life (Ware, Sherbourne. 1992), by a brief, multifunctional assessment of the subject's health. The SF-36 consists of 36 items, including physical function, role-physical, bodily pain, general health, vitality, social function, role-emotional, and mental health. Scores range from 0 to 100, with higher scores representing better physical function and/or health.

### 3.6.5 Clinical Recurrence

Subjects who meet the following criteria during subcutaneous administration in the maintenance treatment phase or in the expansion phase is considered as having a clinical recurrence:
- An increase from maintenance baseline (first subcutaneous dose in the expansion phase) in the partial Mayo score by at least 2 points, with absolute partial Mayo score ≥ 4 points; or
- An absolute partial Mayo score ≥ 7 points. Subjects with clinical recurrence may be given rescue therapy.

### 3.7 Safety Assessment

Laboratory tests: including hematology, blood biochemistry, urinalysis, viral serology, tuberculosis, pregnancy test, Clostridium difficile test, etc.

Clinical examination: including physical examination, vital sign examination, 12-lead electrocardiogram (ECG), severe allergic reaction, infusion reaction, partial reaction of injection, cardiovascular event, psychiatric related events, etc.

### 3.8 Pharmacokinetic Analysis

3.5 mL of whole blood was collected, and the serum was separated, aliquoted, and cryopreserved for PK analysis. The concentration of the antibody in the serum was determined by ELISA. Descriptive statistical analysis was performed on PK parameters, including mean, standard deviation, maximum value, minimum value, median, etc., including but not limited to Cₘₐₓ, AUC, V, T_{1/2} and CL.

### 3.9 Immunogenicity Analysis

About 5 mL of whole blood was collected, and the serum was separated, aliquoted, and cryopreserved for ADA and NAb analysis. If necessary, immunogenicity samples will be used for antibody concentration testing. The positive rates of anti-drug antibody (ADA) and neutralizing antibody (NAb) were summarized and calculated by treatment groups.

### 3.10 Study results

To date, 141 subjects have been enrolled in this study, wherein 94 subjects received 200 mg or 600 mg of the antibody and 47 subjects received placebo during the induction treatment phase (the ratio of subjects in the 200 mg group: 600 mg group: placebo group was 1:1:1). At the end of the induction phase (Week 12), 72 subjects achieved a clinical response and 19 subjects achieved a clinical remission. No significant or unacceptable toxic and side- effects were observed in the enrolled subjects, and the compliance of the patients was good.

The above blinded data of this study were estimated with reference to the compliance rate of other products in the placebo group of the same population study. Assuming that the proportion of subjects achieved a clinical response in the placebo group was 29.9%, the number of subjects achieving a clinical response in the placebo group was 47 × 29.9% = 14. The number of subjects achieving a clinical response in the antibody treatment groups (including 200 mg and 600 mg) was then 72-14 = 58 (i.e., the total number of subjects achieving clinical response minus the number of subjects achieving a clinical response in the placebo group). Thus, the proportion of subjects achieving a clinical response in the antibody treatment group was 58/72 = 61.7%. This proportion was similar to the clinical response proportion of another anti-IL-23p19 subunit antibody, Guselkumab, at the end of the induction phase. Similarly, the proportion of subjects in the antibody treatment groups (including 200 mg and 600 mg) who achieved clinical remission was estimated to be 16.0%.

The blinded data of this study shown that the IL-23p19 subunit antibody administered according to the administration regimen of the present invention can produce a positive therapeutic effect on ulcerative colitis, particularly moderate to severe ulcerative colitis, with good safety and good patient compliance.

The exemplary embodiments of the present invention have been described above, and it should be understood by those skilled in the art that these disclosures are only exemplary and that various other substitutions, adaptations and modifications can be made within the scope of the present invention. Accordingly, the invention is not limited to the specific embodiments set forth herein.

## Claims

1. A method for preventing or treating inflammatory bowel disease, preferably ulcerative colitis or Crohn's disease, comprising administering to a patient in need thereof an anti-IL-23p19 antibody, wherein the administration regimen comprises:
a) an induction treatment phase: administering to the patient in need thereof one or more induction doses of the anti-IL-23p19 antibody, wherein each the induction doses is 100-1000 mg; and
b) a maintenance treatment phase: administering to the patient one or more maintenance doses of the anti-IL-23p19 antibody, the first maintenance dose being administered 2-8 weeks after the last induction dose is administered, wherein each maintenance doses is 100-1000 mg;
wherein the amino acid sequence of the heavy chain CDR1 of the antibody comprises or consists of SEQ ID NO. 1, the amino acid sequence of the heavy chain CDR2 comprises or consists of SEQ ID NO. 2, and the amino acid sequence of the heavy chain CDR3 comprises or consists of SEQ ID NO. 3; the amino acid sequence of the light chain CDR1 of the antibody comprises or consists of SEQ ID NO. 4, the amino acid sequence of the light chain CDR2 comprises or consists of SEQ ID NO. 5, and the amino acid sequence of the light chain CDR3 comprises or consists of SEQ ID NO. 6.

2. The method of claim 1, wherein the induction dose is administered by intravenous injection and/or the maintenance dose is administered by subcutaneous injection.

3. The method of claim 1 or 2, wherein the patient enters the maintenance treatment phase once administration of the induction dose has produced a clinical response.

4. The method of any one of claims 1-3, wherein the induction dose is administered 1-3 times, preferably 3 times.

5. The method of any one of claims 1-4, wherein when multiple induction doses are administered, the induction doses are administered at 4-week intervals; and/or when multiple maintenance doses are administered, the maintenance doses are administered at 4-8 weeks intervals.

6. The method of any one of claims 1-5, wherein the first maintenance dose is administered 2-8 weeks, preferably 4 weeks, after administration of the last induction dose.

7. The method of any one of claims 1-6, wherein the method is for preventing or treating ulcerative colitis (UC), the method comprising administering to the patient in need thereof an anti-IL-23p19 antibody, wherein the administration regimen comprises:
a) an induction treatment phase: administering to the patient in need thereof 3 induction doses of an anti-IL-23p19 antibody at 4-week intervals, wherein each induction dose is 100-1000 mg; and
b) a maintenance treatment phase: administering to the patient maintenance doses of the anti-IL-23p19 antibody at intervals of 4-8 weeks, the first maintenance dose being administered 2-8 weeks after the last induction dose, wherein each maintenance dose is 100-1000 mg;
wherein the amino acid sequence of the heavy chain CDR1 of the antibody comprises or consists of SEQ ID NO. 1, the amino acid sequence of the heavy chain CDR2 comprises or consists of SEQ ID NO. 2, and the amino acid sequence of the heavy chain CDR3 comprises or consists of SEQ ID NO. 3; the amino acid sequence of the light chain CDR1 of the antibody comprises or consists of SEQ ID NO. 4, the amino acid sequence of the light chain CDR2 comprises or consists of SEQ ID NO. 5, and the amino acid sequence of the light chain CDR3 comprises or consists of SEQ ID NO. 6.

8. The method according to claim 7, wherein the UC is moderate to severe ulcerative colitis.

9. The method of claim 7 or 8, wherein the induction dose is administered by intravenous injection, and/or the maintenance dose is administered by subcutaneous injection.

10. The method according to any one of claims 1-9, wherein, in the induction treatment phase, 3 induction doses of the anti-IL-23p19 antibody are administered to the patient in need thereof by intravenous infusion at intervals of 4 weeks.

11. The method according to any one of claims 1-10, wherein in the maintenance treatment phase, a maintenance dose of the anti-IL-23p19 antibody is administered to the patient in need thereof by subcutaneous injection at an interval of 4 weeks or 8 weeks.

12. The method of any one of claims 1-11, wherein the induction dose administered is 100 mg, 200 mg, 300 mg, 400 mg, 500 mg, 600 mg, 700 mg, 800 mg, 900 mg, or 1000 mg, or a range consisting of any two thereof.

13. The method according to any one of claims 1-11, wherein each induction dose is 200 mg.

14. The method according to any one of claims 1-11, wherein each induction dose is 600 mg.

15. The method of any one of claims 1-14, wherein the maintenance dose is administered at an interval of 4 weeks or 8 weeks after administration of the first maintenance dose.

16. The method of any one of claims 1-15, wherein the first maintenance dose is for administration 4 weeks after administration of the last induction dose.

17. The method of any one of claims 1-16, wherein each maintenance dose is 100 mg, 200 mg, 300 mg, 400 mg, 500 mg, 600 mg, 700 mg, 800 mg, 900 mg, or 1000 mg, or a range consisting of any two thereof.

18. The method of any one of claims 1-16, wherein each maintenance dose is 200 mg.

19. The method of any one of claims 1-18, wherein the administration regimen comprises:
a) an induction treatment phase: administering to the patient in need thereof 3 induction doses of the anti-IL-23p19 antibody by intravenous infusion at 4-week intervals, each induction dose being 200 mg or 600 mg; and
b) a maintenance treatment phase: administering maintenance doses of the anti-IL-23p19 antibody to the patient by subcutaneous injection at an interval of 4 weeks or 8 weeks, the first maintenance dose being administered 4 weeks after the last induction dose is administered, each maintenance dose being 200 mg, the administration continuing until Week 44.

20. The method of any one of claims 1-9, wherein the administration regimen comprises an extended induction phase that occurs after the induction treatment phase and before the maintenance treatment phase:
extended induction phase: the patient is administered one or more extended induction doses of the anti-IL-23p19 antibody, wherein the extended induction dose is 100-1000 mg; preferably, the patient enters the maintenance treatment phase once the administration of the extended induction dose has produced a clinical response.

21. The method of claim 20, wherein the extended induction dose is administered by intravenous injection.

22. The method of claim 20 or 21, wherein the extended induction dose is administered 1-3 times, preferably 3 times.

23. The method of any one of claims 20-22, wherein when multiple extended induction doses are administered, the extended induction doses are administered at 4-week intervals.

24. The method according to any one of claims 1-23, wherein the administration regimen further comprises an extended induction phase: after 3 induction doses of the anti-IL-23p19 antibody are administered to the patient in need thereof at 4-week intervals, if the patient does not achieve a clinical response 2-8 weeks after the last induction dose is administered, 3 extended induction doses are administered to the patient, each extended induction dose being 100-1000 mg; if the patient produces a clinical response to the administration of the extended induction dose, the maintenance treatment phase is entered.

25. The method of claim 24, wherein 4 weeks after the last induction dose is administered, if the patient does not achieve a clinical response, 3 extended induction doses are administered to the patient, each extended induction dose being 100-1000 mg; if the patient achieves a clinical response to the administration of the extended induction dose, the maintenance treatment phase is entered.

26. The method of any one of claims 20-25, wherein the first extended induction dose is administered 4 weeks after administration of the last induction dose of the induction treatment phase.

27. The method of any one of claims 20-26, wherein each extended induction dose is 100 mg, 200 mg, 300 mg, 400 mg, 500 mg, 600 mg, 700 mg, 800 mg, 900 mg, or 1000 mg, or a range consisting of any two thereof.

28. The method of claim 27, wherein each extended induction dose is 600 mg.

29. The method of any one of claims 20-28, after the extended induction phase, the first maintenance dose is administered 2-8 weeks after the last extended induction dose is administered.

30. The method of claim 29, after the extended induction phase, the first maintenance dose is administered 4 weeks after the last extended induction dose is administered.

31. The method of any one of claims 20-30, wherein after the extended induction phase, a maintenance doses of the anti-IL-23p19 antibody are administered to the patient in need thereof by subcutaneous injection at 4-week intervals, wherein each maintenance dose is 100-1000 mg.

32. The method of any one of claims 20-31, after the extended induction phase, the maintenance dose is administered at 100 mg, 200 mg, 300 mg, 400 mg, 500 mg, 600 mg, 700 mg, 800 mg, 900 mg, or 1000 mg, or a range consisting of any two thereof.

33. The method of claim 32, wherein each maintenance dose is 200 mg.

34. The method of any one of claims 1-33, wherein the administration regimen comprises:
a) an induction treatment phase: administering to the patient in need thereof 3 induction doses of the anti-IL-23p19 antibody by intravenous infusion at 4-week intervals, each induction dose being 200 mg or 600 mg; and
a1) an extended induction phase: administering to the patient in need thereof 3 extended induction doses of the anti-IL-23p19 antibody by intravenous infusion at 4-week intervals, each extended induction dose being 600 mg; and
b) a maintenance treatment phase: administering to the patient maintenance doses of the anti-IL-23p19 antibody by subcutaneous injection at an interval of 4 weeks or 8 weeks, the first maintenance dose being administered 4 weeks after the last extended induction dose is administered, each maintenance dose being 200 mg, the administration continuing until Week 56.

35. The method according to any one of claims 1-34, wherein the heavy chain of the anti-IL-23p19 antibody comprises an N-glycosylation site located at asparagine 295 of the heavy chain.

36. The method according to any one of claims 1-35, wherein the anti-IL-23p19 antibody comprises a heavy chain variable region VH and a light chain variable region VL, the heavy chain variable region comprises or consists of the amino acid sequences of SEQ ID NOs:7 or a sequence having at least 90%, 95%, 98% or 99% identity thereto, and the light chain variable region comprises or consists of SEQ ID NO:8 or a sequence having at least 90%, 95%, 98% or 99% identity thereto.

37. The method according to any one of claims 1-36, wherein the anti-IL-23p19 antibody is an IgG1 antibody, preferably comprising a heavy chain and a light chain, wherein the heavy chain comprises or consists of SEQ ID NO: 9 or a sequence having at least 90%, 95%, 98%, or 99% identity thereto, and the light chain comprises or consists of SEQ ID NO: 10 or a sequence having at least 90%, 95%, 98% or 99% identity thereto.

38. The method according to any one of claims 1-37, wherein the anti-IL-23p19 antibody comprises a heavy chain set forth in SEQ ID NO: 9 and a light chain set forth in SEQ ID NO: 10; for example, the anti-IL-23p19 antibody comprises or consists of 2 heavy chains set forth in SEQ ID NO: 9 and 2 light chains set forth in SEQ ID NO: 10.

39. The method according to any one of claims 1-38, wherein the anti-IL-23p19 antibody is recombinantly expressed in HEK 293 cells or CHO cells.

40. An anti-IL-23p19 antibody for use in the prevention or treatment of inflammatory bowel disease, e.g., ulcerative colitis or Crohn's disease, wherein the amino acid sequence of the heavy chain CDR1 of the antibody comprises or consists of SEQ ID NO. 1, the amino acid sequence of the heavy chain CDR2 comprises or consists of SEQ ID NO. 2, and the amino acid sequence of the heavy chain CDR3 comprises or consists of SEQ ID NO. 3; the amino acid sequence of the light chain CDR1 of the antibody comprises or consists of SEQ ID NO. 4, the amino acid sequence of the light chain CDR2 comprises or consists of SEQ ID NO. 5, and the amino acid sequence of the light chain CDR3 comprises or consists of SEQ ID NO. 6, and wherein the administration regimen of the antibody comprises:
a) an induction treatment phase: administering one or more induction doses of the anti-IL-23p19 antibody to the patient in need thereof, wherein each the induction doses is 100-1000 mg; and
b) a maintenance treatment phase: administering to the patient one or more maintenance doses of the anti-IL-23p19 antibody, the first maintenance dose being administered 2-8 weeks after the last induction dose is administered, wherein each maintenance doses is 100-1000 mg.

41. The anti-IL-23p19 antibody for use according to claim 40, wherein the administration regimen is as defined in any one of claims 2-7 and 9-34, and/or wherein the anti-IL-23p19 antibody is as defined in any one of claims 35-39.

42. Use of an anti-IL-23p19 antibody in the manufacture of a medicament for preventing or treating inflammatory bowel disease, e.g., ulcerative colitis or Crohn's disease, wherein the amino acid sequence of the heavy chain CDR1 of the antibody comprises or consists of SEQ ID NO. 1, the amino acid sequence of the heavy chain CDR2 comprises or consists of SEQ ID NO. 2, and the amino acid sequence of the heavy chain CDR3 comprises or consists of SEQ ID NO. 3; the amino acid sequence of the light chain CDR1 of the antibody comprises or consists of SEQ ID NO. 4, the amino acid sequence of the light chain CDR2 comprises or consists of SEQ ID NO. 5, and the amino acid sequence of the light chain CDR3 comprises or consists of SEQ ID NO. 6, and wherein the administration regimen of the antibody or the medicament comprises:
a) an induction treatment phase: administering one or more induction doses of the anti-IL-23p19 antibody to the patient in need thereof, wherein each the induction doses is 100-1000 mg;
b) a maintenance treatment phase: administering to the patient one or more maintenance doses of the anti-IL-23p19 antibody, the first maintenance dose being administered 2-8 weeks after the last induction dose is administered, wherein each the maintenance doses is 100-1000 mg.

43. The use according to claim 41, wherein the administration regimen is as defined in any one of claims 2-7 and 9-34, and/or wherein the anti-IL-23p19 antibody is as defined in any one of claims 35-39.

44. A kit of parts, comprising an effective amount of the anti-IL-23p19 antibody as defined in any one of claims 1-39, and preferably further comprising a package insert with instructions for using the anti-IL-23p19 antibody to prevent or treat inflammatory bowel disease, e.g., ulcerative colitis or Crohn's disease, in an individual.

45. Use of the kit of parts according to claim 44 in the manufacture of a medicament for preventing or treating inflammatory bowel disease, such as ulcerative colitis or Crohn's disease.
